# EUROPEAN PATENT SPECIFICATION

(11) **EP 3 801 563 B1**
(45) Date of publication and mention of the grant of the patent: **12.07.2023**
(21) Application number: 19812602.1
(22) Date of filing: 31.05.2019
(51) Int. Cl.: A61K 39/00, A61P 35/02, C12N 5/10

(54) **MATERIALS AND METHODS FOR TREATING CANCER**
MATERIALIEN UND VERFAHREN ZUR BEHANDLUNG VON KREBS
MATÉRIELS ET PROCÉDÉS DE TRAITEMENT DU CANCER

(30) Priority: 01.06.2018 US 201862679348 P; 31.10.2018 US 201862753485 P
(43) Date of publication of application: 14.04.2021
(73) Proprietor: Mayo Foundation for Medical Education and Research, Rochester, Minnesota 55905 (US)
(72) Inventor: KENDERIAN, Saad J., Rochester, Minnesota 55902 (US); STERNER, Rosalie M., Claremont, Minnesota 55924-4560 (US); COX, Michelle J., Rochester, Minnesota 55901-3180 (US); SAKEMURA, Reona, Rochester, Minnesota 55901 (US)
(74) Representative: Pearl Cohen Zedek Latzer Baratz UK LLP
(86) International application number: PCT/US2019/034900
(87) International publication number: WO 2019/232370

(56) References cited:
- WO-A1-2014/011984
- WO-A1-2015/066262
- STERNER R M ET AL: "GM-CSF Blockade during Chimeric Antigen Receptor T Cell Therapy Reduces Cytokine Release Syndrome and Neurotoxicity and May Enhance Their Effector Functions", BLOOD, vol. 132, no. Suppl. 1, 961, 29 November 2018 (2018-11-29), XP086593679, 60th Annual Meeting of the American Society of Hematology; San Diego, CA, USA; 1-4 December 2018 ISSN: 0006-4971, DOI: 10.1182/BLOOD-2018-99-111766
- STERNER R M ET AL: "GM-CSF inhibition reduces cytokine release syndrome and neuroinflammation but enhances CAR-T cell function in xenografts", BLOOD, vol. 133, no. 7, 14 February 2019 (2019-02-14), pages 697-709, XP055613201, ISSN: 0006-4971, DOI: 10.1182/blood-2018-10-881722
- SACHDEVA M ET AL: "Granulocyte-macrophage colony-stimulating factor inactivation in CAR T-cells prevents monocyte-dependent release of key cytokine release syndrome mediators", JOURNAL OF BIOLOGICAL CHEMISTRY, vol. 294, no. 14, 25 February 2019 (2019-02-25), pages 5430-5437, XP055639281, ISSN: 0021-9258, DOI: 10.1074/jbc.AC119.007558
- STERNER R M ET AL: "Using CRISPR/Cas9 to Knock Out GM-CSF in CAR-T Cells", JOURNAL OF VISUALIZED EXPERIMENTS, vol. 149, E59629, 22 July 2019 (2019-07-22), XP009528961, ISSN: 1940-087X, DOI: 10.3791/59629
- COX M J ET AL: "Improved Anti-Tumor Response of Chimeric Antigen Receptor T Cell (CART) Therapy after GM-CSF Inhibition Is Mechanistically Supported By a Novel Direct Interaction of GM-CSF with Activated Carts", BLOOD, vol. 134, no. Suppl. 1, 3868, 13 November 2019 (2019-11-13), XP086673057, 61st Annual Meeting and Exposition of the American Society of Hematology; Orlando, FL, USA; 7-10 December 2019 ISSN: 0006-4971, DOI: 10.1182/BLOOD-2019-129349

## Description

### BACKGROUND

### 1. Technical Field

This document relates to materials involved in treating cancer. For example, this document provides methods for making chimeric antigen receptor T cells having reduced expression levels of one or more cytokines (e.g., GM-CSF) and their use in adoptive cell therapy (*e.g.,* a chimeric antigen receptor T cell therapy) to treat a mammal (*e.g.,* a human) having cancer.

### 2. Background Information

Unprecedented results from pivotal trials evaluating the safety and efficacy of CD19 directed chimeric antigen receptor T cells (CART19) have led to the recent FDA approval of CART19 (Tisagenlecleucel) for relapsed refractory acute lymphoblastic leukemia (ALL) and CART19 (Axi-Cel) for the treatment of diffuse large B cell lymphoma (DLBCL). The application of CART cell therapy is associated with toxicities resulting in cytokine release syndrome (CRS) and neurotoxicity. Additionally, the efficacy of CART cell therapy is limited to only 40% durable remissions in lymphoma and 50-60% durable remissions in acute leukemia.

WO 2015/066262 discloses methods for preventing or reducing toxicity of adoptive cell therapy using a population of cells deficient in the expression or activity of GM-CSF, GM-CSF receptor or perforin.

WO 2014/011984 discloses treating cancer in a patient by a first-line therapy comprising administering to a patient a genetically modified T cell expressing a CAR wherein the CAR comprises an antigen binding domain, a transmembrane domain, a costimulatory signaling region, and a CD3 zeta signaling domain, and monitoring the levels of cytokines in the patient post T cell infusion to determine the appropriate type of second-line of therapy for the patient as a consequence of the presence of the CAR T cell in the patient.

### SUMMARY

The present invention provides a method for making a chimeric antigen receptor T cell having a reduced level of granulocyte-macrophage colony-stimulating factor (GM-CSF) polypeptides, the method comprising:
introducing a nucleic acid construct into a T cell ex vivo, wherein the nucleic acid construct comprises: a) a nucleic acid encoding a guide RNA, wherein the guide RNA is complementary to a GM-CSF messenger RNA, b) a nucleic acid encoding a Cas nuclease, and c) a nucleic acid encoding the chimeric antigen receptor,
wherein the nucleic acid encoding the chimeric antigen receptor comprises the nucleic acid sequence set forth in SEQ ID NO: 2 and wherein the chimeric antigen receptor targets CD19 tumor-associated antigen.

In an embodiment, the guide RNA comprises a nucleic acid sequence set forth in SEQ ID NO: 1.

In an embodiment, the Cas nuclease is Cas9 nuclease.

In an embodiment, the nucleic acid construct is a viral vector. In an embodiment, the viral vector is a lentiviral vector.

In an embodiment, the introducing step comprises transduction.

In an embodiment, the chimeric antigen receptor (CAR) T cells (CARTs) have a reduced expression level of GM-CSF polypeptides. For example, a T cell (*e*.*g*., a CART) can be engineered to have reduced GM-CSF polypeptide expression (*e*.*g*., for use in adoptive cell therapy). In some cases, a T cell (*e.g.,* a CART) can be engineered to knock out (KO) a nucleic acid encoding a GM-CSF polypeptide to reduce GM-CSF polypeptide expression in that T cell. T cells (*e.g.,* CARTs) having a reduced level of GM-CSF polypeptides can be administered (*e*.*g*., in an adoptive cell therapy) to a mammal having cancer to treat the mammal.

As demonstrated herein, GM-CSF KO CARTs produce reduced levels of GM-CSF and continue to function normally in both *in vitro* and *in vivo* models. Also as demonstrated herein, GM-CSF KO CARTs can enhance CART cell function and antitumor activity. For example, enhanced CART cell proliferation and antitumor activity can be observed after GM-CSF depletion. CART19 antigen specific proliferation in the presence of monocytes can be increased *in vitro* after GM-CSF depletion. In ALL patient derived xenografts, CART19 cells can result in a more durable disease control when combined with lenzilumab, and GM-CSF^{k/o} CART19 cells can be more effective in controlling leukemia in NALM6 xenografts. In some cases, GM-CSF KO CARTs can be incorporated into adoptive T cell therapies (e.g., CART cell therapies), for example, for use in treating mammals having cancer without resulting in CRS and/or neurotoxicity. For example, GM-CSF KO CARTs can be incorporated into adoptive T cell therapies (e.g., CART cell therapies) to enhance the therapeutic window after CART cell therapy. In some cases, a single construct can be used both to introduce a CAR into a cell (e.g., a T cell) and to reduce or knock out expression of one or more cytokine polypeptides in that same cell.

The present invention also provides a chimeric antigen receptor T cell having a reduced level of granulocyte-macrophage colony-stimulating factor (GM-CSF) polypeptides for use in the treatment of cancer, wherein the chimeric antigen receptor T cell comprises a chimeric antigen receptor encoded by the nucleic acid sequence set forth in SEQ ID NO: 2 and wherein the chimeric antigen receptor targets CD19 tumor-associated antigen.

In an embodiment, the cancer is a lymphoma. In an embodiment, the lymphoma is a diffuse large B cell lymphoma.

In an embodiment, the cancer is a leukemia. In an embodiment, the leukemia is an acute lymphoblastic leukemia.

The present invention further provides a method for improving T cell effector functions of a chimeric antigen receptor T cell, the method comprising:
introducing a nucleic acid construct into a T cell ex vivo, wherein the nucleic acid construct comprises: a) a nucleic acid encoding a guide RNA, wherein the guide RNA is complementary to a GM-CSF messenger RNA b) a nucleic acid encoding a Cas nuclease, and c) a nucleic acid encoding the chimeric antigen receptor,
wherein the nucleic acid encoding the chimeric antigen receptor comprises the nucleic acid sequence set forth in SEQ ID NO:2 and wherein the chimeric antigen receptor targets CD19 tumor-associated antigen.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

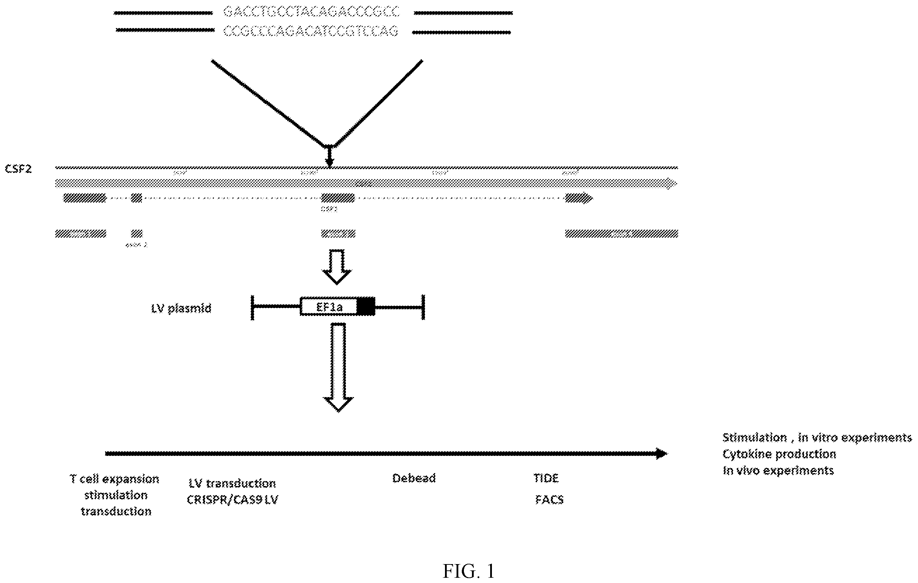

Figure 1 contains a schematic of an exemplary method of using CRISPR to engineer a GM-CSF knock out (KO) cell. Guide RNA (GACCTGCCTACAGACCCGCC; SEQ ID NO:1) targeting exon 3 of GM-CSF (also known as colony-stimulating factor 2 (CSF2)) was synthesized and cloned into a lentivirus (LV) plasmid. This LV plasmid was used to transduce 293T cells and lentivirus particles were collected at 24 hours and 48 hours and were concentrated. To generate GM-CSF knocked out CART cells, T cells were stimulated with CD3/CD28 beads on day 0. On day 1, T cells were transduced with CAR19 lentivirus particles, and simultaneously with GMCSF knockout CRISPR/Cas9 lentivirus particles. T cells were expanded for 8 days and then harvested.

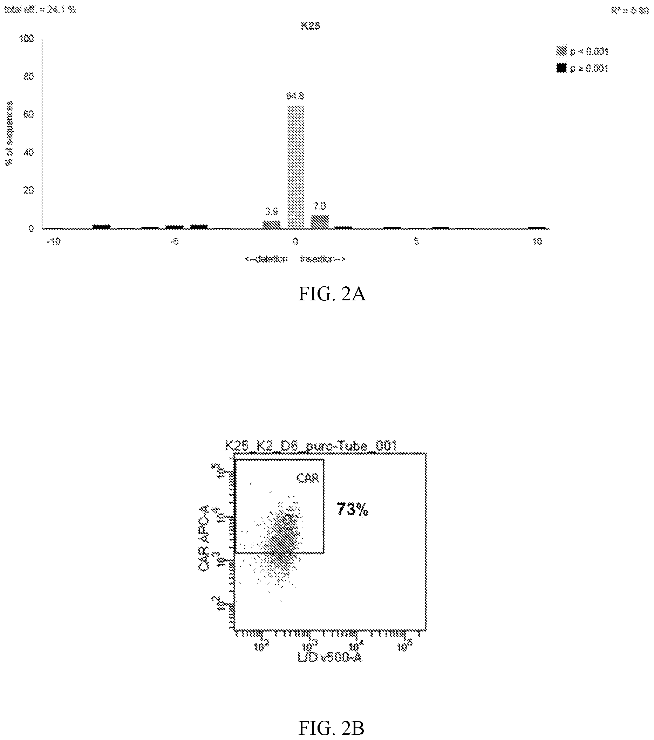

Figures 2A and 2B show CAR transduction and GM-CSF knockout efficiency. Figure 2A contains a graph showing that CRISPR/Cas9 lentivirus with a guide RNA directed to exon 3 of GM-CSF resulted in a knockout efficiency of 24.1%. At the end of the expansion, CART cells were harvested, and DNA was isolated and sent for sequencing to be compared to control sequences. This yielded in a knockout efficiency of 24.1%. Figure 2B contains a flow cytometric analysis showing that CAR transduction efficiency after transduction with lentivirus was 73%. Flow cytometric analysis was performed on Day 6 after lentivirus transduction.

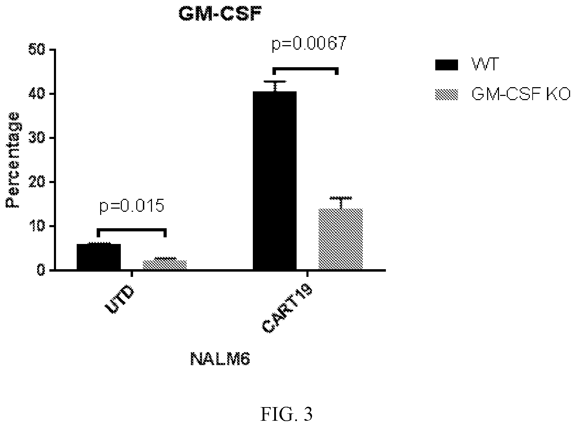

Figure 3 shows that GM-CSF KO CART19 cells produce less GM-CSF compared to CART cells, and GM-CSF knockout control T cells produce less amount of GM-CSF compared to control untransduced T cells (UTD). CART19, GM-CSF KO CART19, UTD, or GM-CSF KO UTD were co-cultured with the CD19 positive cell line NALM6 at a ratio of 1:5. 4 hours later, the cells were harvested, permeabilized, and fixed; and intra-cellular staining for cytokines was performed.

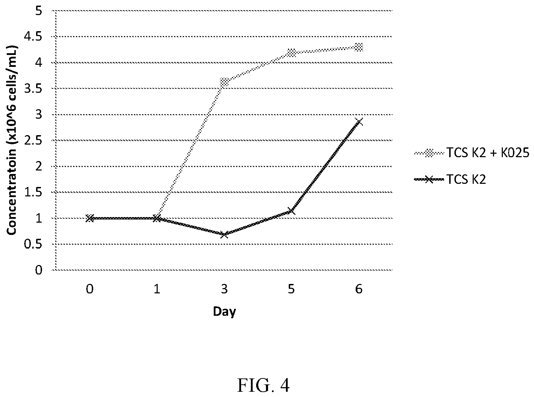

Figure 4 shows that GM-CSF KO CART19 cells expand more robustly compared to CART19. After T cells were transduced with the virus, their expansion kinetics was followed. GM-CSF KO expand more robustly compared to CART19 alone.

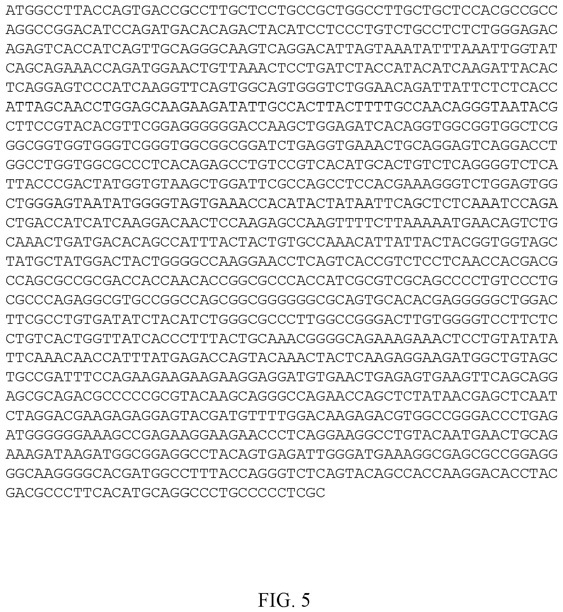

Figure 5 shows an exemplary nucleic acid sequence (SEQ ID NO:2) encoding a CAR targeting CD19 (CAR19).

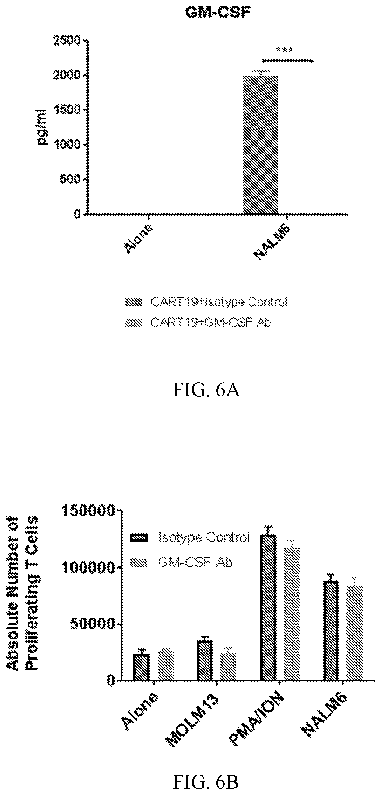

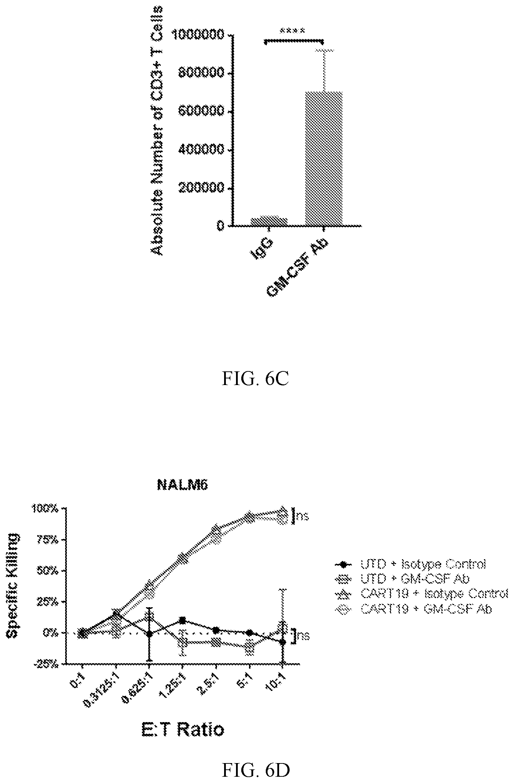

Figures 6A-6D show that GM-CSF neutralization *in vitro* enhances CART cell proliferation in the presence of monocytes and does not impair CART cell effector function. Figure 6A contains a graph showing that lenzilumab neutralizes CART cell produced GM-CSF *in vitro* compared to isotype control treatment as assayed by multiplex after 3 days of culture with CART19 in media alone or CART19 co-cultured with NALM6, n=2 experiments, 2 replicates per experiment, representative experiment depicted, *** p<0.001 between lenzilumab and isotype control treatment, t test, mean±SEM. Figure 6B contains a graph showing that GM-CSF neutralizing antibody treatment did not inhibit the ability of CART cells to proliferate as assayed by CSFE flow cytometry proliferation assay of live CD3 cells, n=2 experiments, 2 replicates per experiment, representative experiment at 3 day time point depicted, ns p>0.05 between lenzilumab and isotype control treatment, t test, mean±SEM. Alone: CART19 in media alone, MOLM13: CART19+MOLM13, PMA/ION: CART19+5ng/mL PMA/0.1ug/mL ION, NALM6: CART19+NALM6. Figure 6C contains a graph showing that lenzilumab enhanced the proliferation of CART19 compared to isotype control treated with CART19 when co-cultured with monocytes n=3 biologic replicates at 3 day time point, 2 replicates per biological replicate, **** p<0.0001, mean±SEM. Figure 6D contains a graphs showing that lenzilumab treatment did not inhibit cytotoxicity of CART19 or untransduced T cells (UTD) when cultured with NALM6, n=2 experiments, 2 replicates per experiment, representative experiment at 48hr time point depicted, ns p>0.05 between lenzilumab and isotype control treatment, t test, mean±SEM.

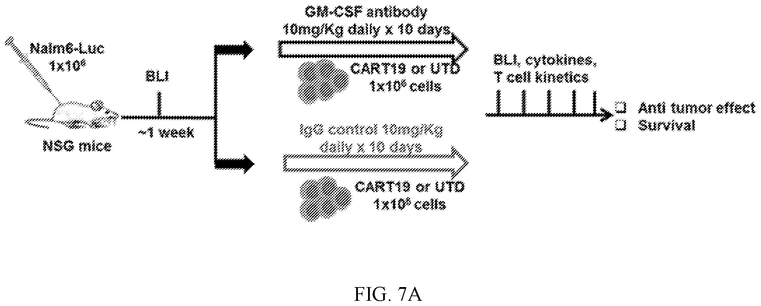

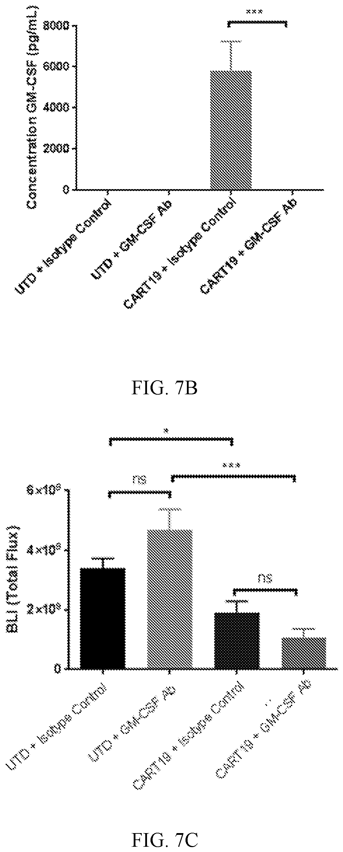

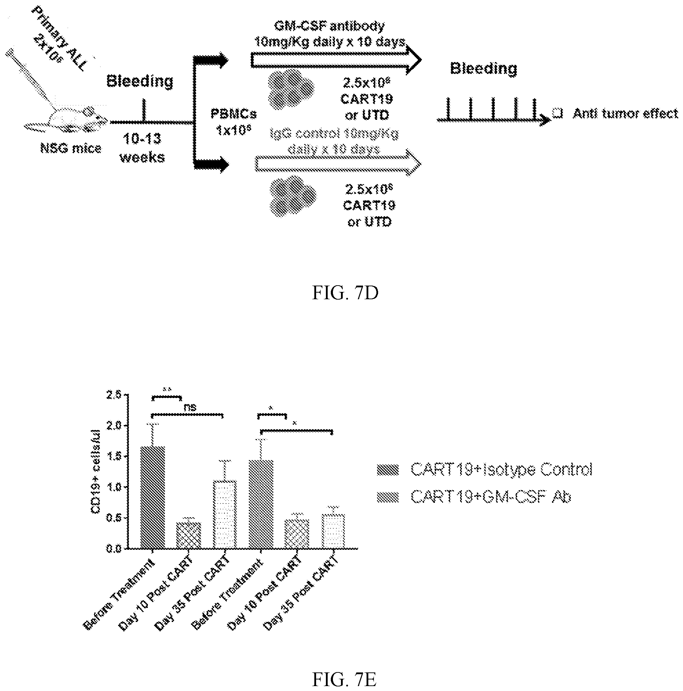

Figures 7A-7E show that GM-CSF neutralization *in vivo* enhances CART cell anti-tumor activity in xenograft models. Figure 7A contains an experimental schema showing that NSG mice were injected with the CD19+ luciferase+ cell line NALM6 (1×10⁶ cells per mouse I.V). 4-6 days later, mice were imaged, randomized, and received 1-1.5×10⁶CART19 or equivalent number of total cells of control UTD cells the following day with either lenzilumab or control IgG (10 mg/Kg, given IP daily for 10 days, starting on the day of CART injection). Mice were followed with serial bioluminescence imaging to assess disease burden beginning day 7 post CART cell injection and were followed for overall survival. Tail vein bleeding was performed 7-8 days after CART cell injection. Figure 7B contains a graph showing that lenzilumab neutralizes CART produced serum GM-CSF *in vivo* compared to isotype control treatment as assayed by GM-CSF singleplex, n=2 experiments, 7-8 mice per group, representative experiment, serum from day 8 post CART cell/UTD injection, *** p<0.001 between lenzilumab and isotype control treatment, t test, mean±SEM. Figure 7C contains a graph showing that lenzilumab treated CART *in vivo* are equally effective at controlling tumor burden compared to isotype control treated CART in a high tumor burden relapse xenograft model of ALL, day 7 post CART injection, n=2 experiments, 7-8 mice per group, representative experiment depicted, *** p<0.001, * p<0.05, ns p>0.05, t test, mean±SEM. Figure 7D contains an experimental schema showing that NSG mice were injected with the blasts derived from patients with ALL (1×10⁶ cells per mouse I.V). Mice were bled serially and when the CD19+ cells >1/uL, mice were randomized to receive 5×10⁶ CART19 (transduction efficiency is around 50%) or UTD cells with either lenzilumab or control IgG (10 mg/Kg, given IP daily for 10 days, starting on the day of CART injection). Mice were followed with serial tail vein bleeding to assess disease burden beginning day 14 post CART cell injection and were followed for overall survival. Figure 7E contains a graph showing that lenzilumab treatment with CART therapy results in more sustained control of tumor burden over time in a primary acute lymphoblastic leukemia (ALL) xenograft model compared to isotype control treatment with CART therapy, 6 mice per group, ** p<0.01, * p<0.05, ns p>0.05, t test, mean±SEM.

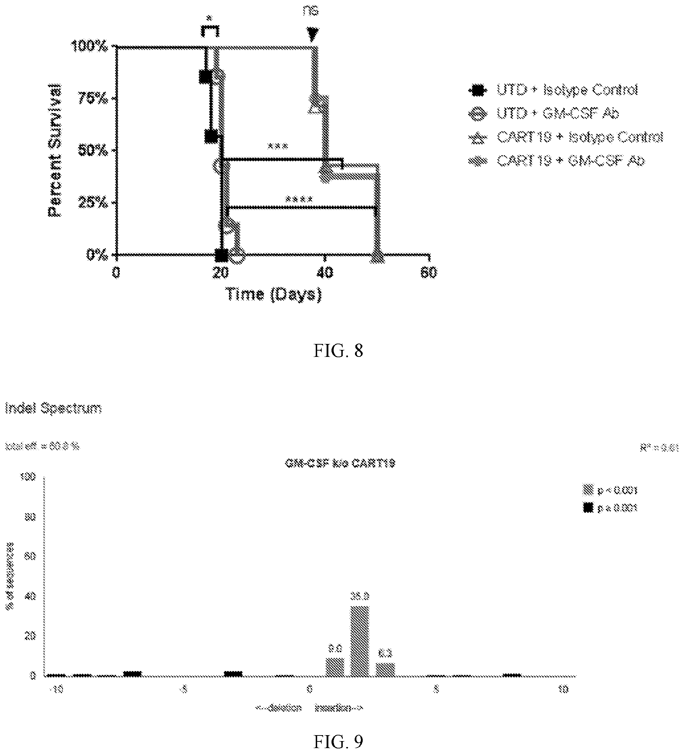

Figure 8 contains a graph showing that lenzilumab+CART cell treated mice have comparable survival compared to isotype control+CART cell treated mice in a high tumor burden relapse xenograft model of ALL. n=2 experiments, 7-8 mice per group, representative experiment depicted, **** p<0.0001, *** p<0.001, * p<0.05, log-rank.
Figure 9 contains a graph showing a representative TIDE sequence to verify genome alteration in the GM-CSF CRISPR Cas9 knockout CART cells. n=2 experiments, representative experiment depicted.

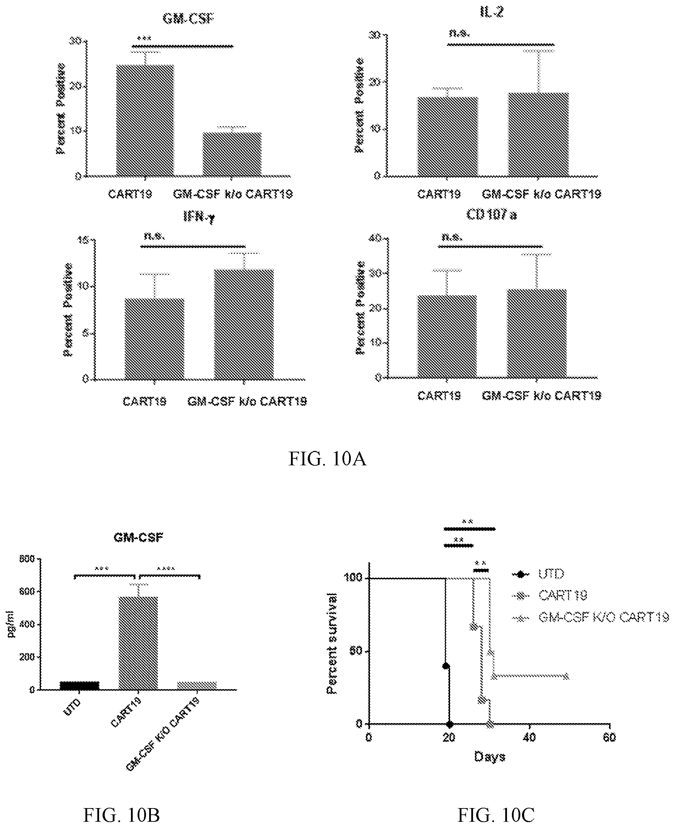

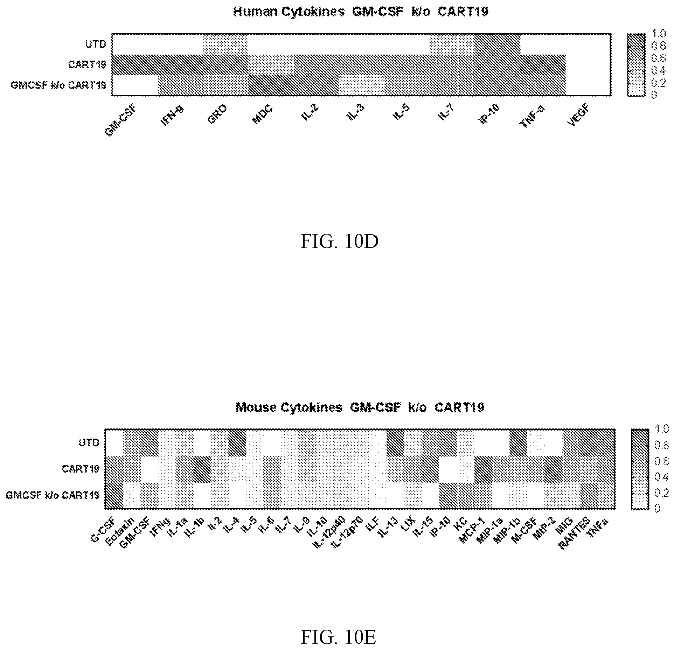

Figures 10A-10E show that GM-CSF CRISPR knockout CART cells exhibit reduced expression of GM-CSF, similar levels of key cytokines, and enhanced anti-tumor activity. Figure 10A contains graphs showing that the CRISPR Cas9 GM-CSF^{k/o} CART exhibit reduced GMCSF production compared to wild type CART19, but other cytokine production and degranulation are not inhibited by the GM-CSF gene disruption, n=3 experiments, 2 replicates per experiment, *** p<0.001, * p<0.05, ns p>0.05 comparing GM-CSF k/o CART and CART, t test, mean±SEM. Figure 10B contains a graph showing that GM-CSF k/o CART have reduced serum human GM-CSF *in vivo* compared to CART treatment as assayed by multiplex, 5-6 mice per group (4-6 at time of bleed, 8 days post CART injection), **** p<0.0001, *** p<0.001 between GM-CSF k/o CART cells and wild type CART cells, t test, mean±SEM. Figure 10C contains a graph showing that GM-CSF^{k/o} CART19 *in vivo* enhances overall survival compared to wild type CART19 in a high tumor burden relapse xenograft model of ALL, 5-6 mice per group, ** p<0.01, log-rank. Figures 10D and 10E contains heat maps showing human (D) and mouse (E) cytokines from multiplex of serum, other than human GM-CSF, show no statistical differences between the GM-CSF k/o CART cells and wild type CART cells, further implicating critical T-cell cytokines aren't adversely depleted by reducing GM-CSF expression, 5-6 mice per group (4-6 at time of bleed), **** p<0.0001, t test.

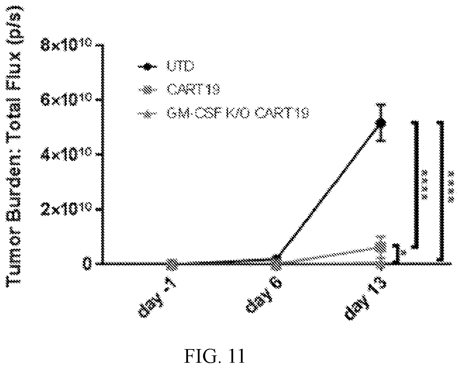

Figure 11 contains a graph showing that GM-CSF knockout CART cells *in vivo* shows slightly enhanced control of tumor burden compared to CART in a high tumor burden relapse xenograft model of ALL. Days post CART injection listed on x-axis, 5-6 mice per group (2 remained in UTD group at day 13), representative experiment depicted, **** p<0.0001, * p<0.05, 2 way ANOVA, mean+SEM.

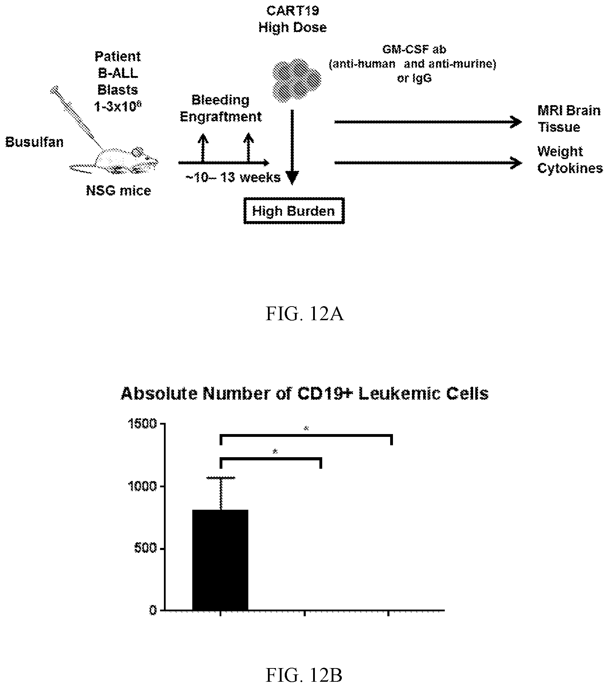

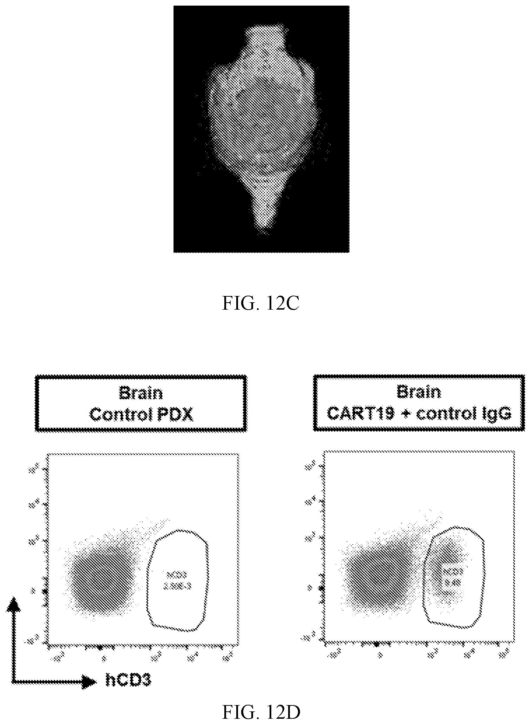

Figures 12A-12D show that patient derived xenograft model for neurotoxicity and cytokine release syndrome. Figure 12A contains an experimental schema showing that mice received 1-3×10⁶ primary blasts derived from the peripheral blood of patients with primary ALL. Mice were monitored for engraftment for -10-13 weeks via tail vein bleeding. When serum CD19⁺ cells were ≥10 cells/uL, the mice received CART19 (2-5×10⁶ cells) and commenced antibody therapy for a total of 10 days, as indicated. Mice were weighed on a daily basis as a measure of their well-being. Mouse brain MRIs were performed 5-6 days post CART19 injection and tail vein bleeding for cytokine and T cell analysis was performed 4-11 days post CART19 injection, 2 independent experiments. Figure 12B contains a graph showing that combination of GM-CSF neutralization with CART19 is equally effective as isotype control antibodies combined with CART19 in controlling CD19+ burden of ALL cells, representative experiment, 3 mice per group, 11 days post CART19 injection, * p<0.05 between GM-CSF neutralization+CART19 and isotype control+CART19, t test, mean±SEM. Figure 12C contains an image showing that brain MRI with CART19 therapy exhibits T1 enhancement, suggestive of brain blood-brain barrier disruption and possible edema. 3 mice per group, 5-6 days post CART19 injection, representative image. Figure 12D contains graphs showing that high tumor burden primary ALL xenografts treated with CART19 show human CD3 cell infiltration of the brain compared to untreated PDX controls. 3 mice per group, representative image.

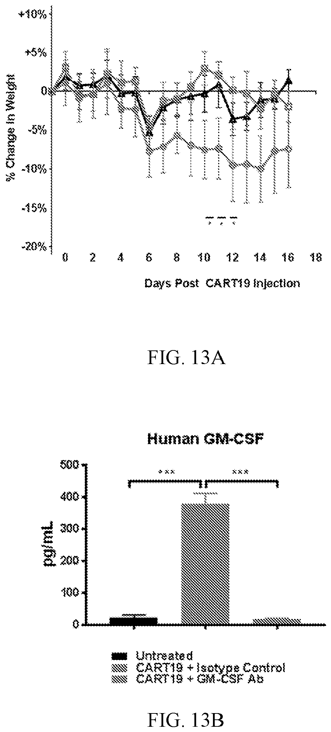

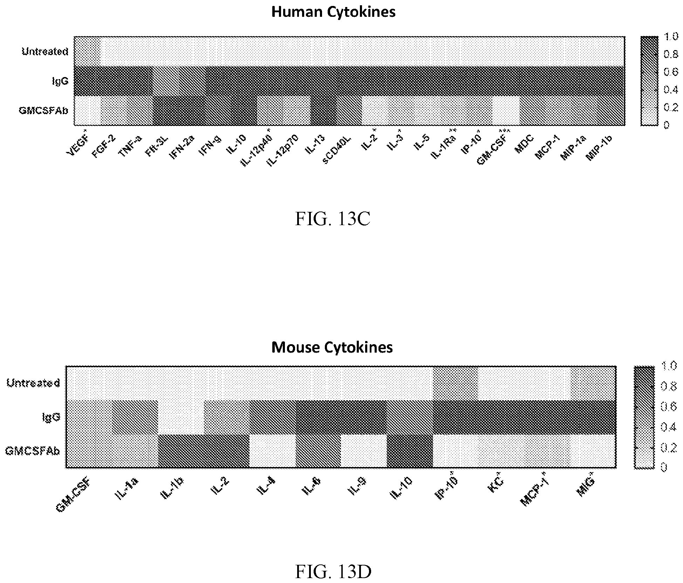

Figures 13A-13D show that GM-CSF neutralization *in vivo* ameliorates cytokine release syndrome after CART19 therapy in a xenograft model. Figure 13A contains a graph showing that lenzilumab & anti-mouse GM-CSF antibody prevent CRS induced weight loss compared to mice treated with CART19 and isotype control antibodies, 3 mice per group, 2 way anova, mean±SEM. Figure 13B contains a graph showing that human GM-CSF was neutralized in patient derived xenografts treated with lenzilumab and mouse GM-CSF neutralizing antibody, 3 mice per group, *** p<0.001, * p<0.05, t test, mean±SEM. Figure 13C contains a heat map showing that human cytokines (serum collected 11 days after CART19 injection) exhibit increase in cytokines typical of CRS after CART19 treatment. GM-CSF neutralization results in significant decrease in several cytokines compared to mice treated with CART19 and isotype control antibodies, including several myeloid associated cytokines, as indicated in the panel, 3 mice per group, serum from day 11 post CART19 injection, *** p<0.001, ** p<0.01, * p<0.05, comparing GM-CSF neutralizing antibody treated and isotype control treated mice that received CART cell therapy, t test. Figure 13D contains a heat map showing that mouse cytokines (serum collected 11 days after CART19 injection) exhibit increase in mouse cytokines typical of CRS after CART19 treatment. GM-CSF neutralization results in significant decrease in several cytokines compared to treated with CART19 with control antibodies, including several myeloid differentiating cytokines, as indicated in the panel, 3 mice per group, serum from day 11 post CART19 injection, * p<0.05, comparing GM-CSF neutralizing antibody treated and isotype control treated mice that received CART cell therapy, t test.

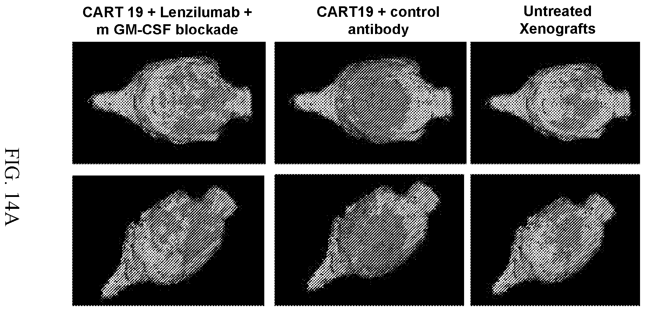

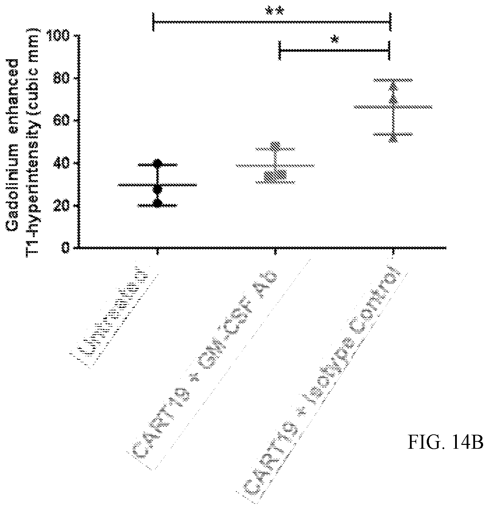

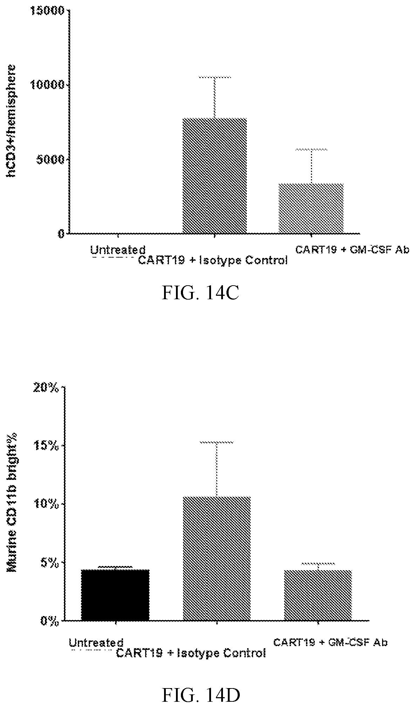

Figures 14A-14D show that GM-CSF neutralization *in vivo* ameliorates neurotoxicity after CART19 therapy in a xenograft model. Figures 14A and 14B show that gadolinium enhanced T1-hyperintensity (cubic mm) MRI showed that GM-CSF neutralization helped reduced brain inflammation, blood-brain barrier disruption, and possible edema compared to isotype control (A) representative images, (B) 3 mice per group, ** p<0.01, * p<0.05, 1 way ANOVA, mean+SD. Figure 14C contains a graph showing that human CD3 T cells were present in the brain after treatment with CART19 therapy. GM-CSF neutralization resulted in a trend toward decreased CD3 infiltration in the brain as assayed by flow cytometry in brain hemispheres, 3 mice per group, mean±SEM. Figure 14D contains a graph showing that CD11b+ bright macrophages were decreased in the brains of mice receiving GM-CSF neutralization during CART therapy compared to isotype control during CART therapy as assayed by flow cytometry in brain hemispheres, 3 mice per group, mean±SEM.

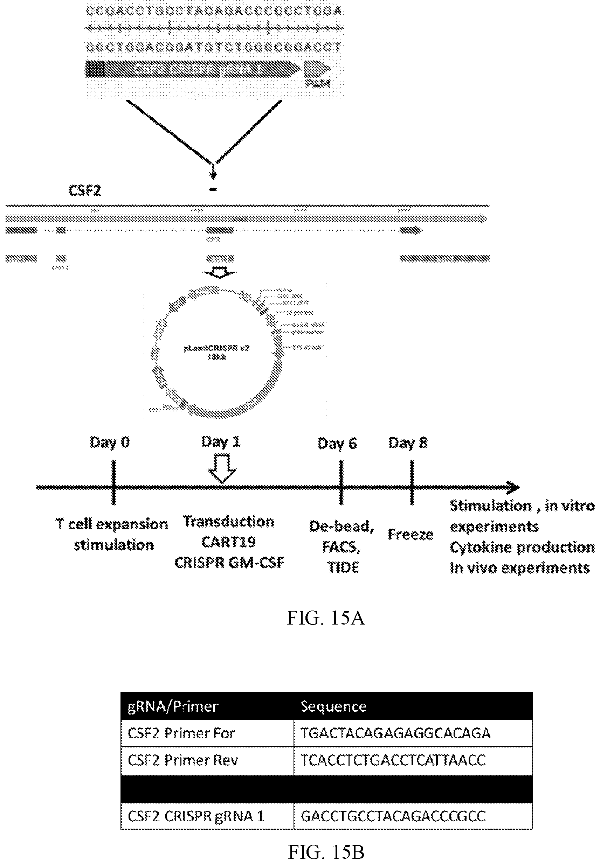

Figures 15A-15B show an exemplary generation of GM-CSFk/o CART19 cells. The experimental schema depicts the schema. Figure 15A shows a gRNA sequence targeting location in CSF2 (GACCTGCCTACAGACCCGCC; SEQ ID NO:11) for generation of GM-CSFk/o CART19. Figure 15B shows primer sequences (TGACTACAGAGAGGCACAGA (SEQ ID NO:12) and TCACCTCTGACCTCATTAACC (SEQ ID NO:13)) and the gRNA sequence (GACCTGCCTACAGACCCGCC; SEQ ID NO:7) used for generation of GM-CSFk/o CART19. To generate GM-CSFk/o CART19 cells, gRNA was clones into a Cas9 lentivirus vector under the control of a U6 promotor and used for lentivirus production. T cells derived from normal donors were stimulated with CD3/CD28 beads and dual transduced with CAR19 virus and CRISPR/Cas9 virus 24 hours later. CD3/CD28 magnetic bead removal was performed on Day +6 and GM-CSFk/o CART19 cells or control CART19 cells were cryopreserved on Day 8.

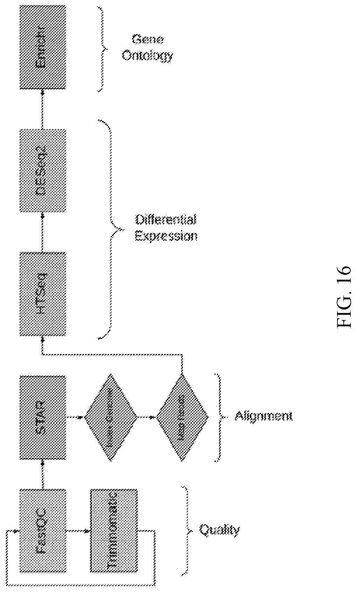

Figure 16 shows a flow chart for procedures used in RNA sequencing. The binary base call data was converted to fastq using Illumina bcl2fastq software. The adapter sequences were removed using Trimmomatic, and FastQC was used to check for quality. The latest human (GRCh38) and mouse (GRCm38) reference genomes were downloaded from NCBI. Genome index files were generated using STAR30, and the paired end reads were mapped to the genome for each condition. HTSeq was used to generate expression counts for each gene, and DeSeq2 was used to calculate differential expression. Gene ontology was assessed using Enrichr.

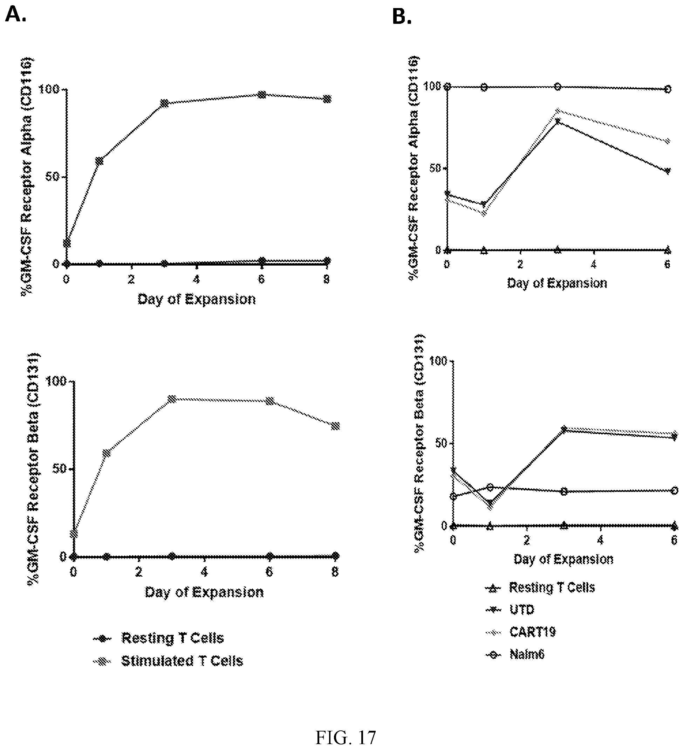

Figures 17A-17B show that GM-CSF receptors are upregulated on T cells and CART cells upon stimulation. Figure 17A shows measurements of CSF2RA (CD116) and CSF2RB (CD131) on T cells versus resting T cells (negative control) during 8-day T cell expansion protocol with CD3/CD28 beads. CSF2RA and CSF2RB expression increased after initial stimulation, peaked on Day 3, and reduced slightly after debeading on Day 6. Figure 17B shows measurements of CSF2RA (CD116) and CSF2RB (CD131) on CART19 and UTD cells versus control during 8-day CART production. Expression decreased slightly on Day 1 and peaked on Day 3.

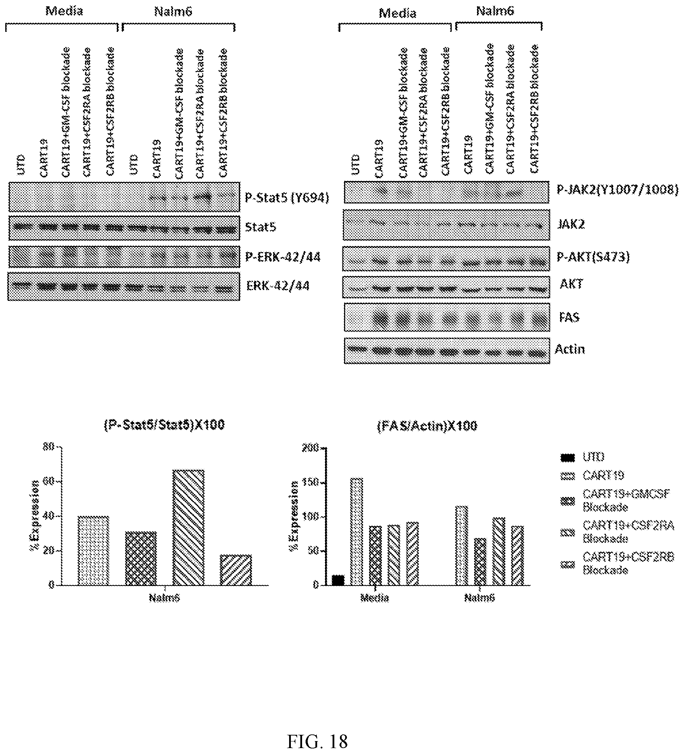

Figure 18 shows that GM-CSF interaction with CSF2 Receptor depends on the beta chain (CSF2RB). Phosphorylated StatS protein expression increased in the presence of irradiated Nalm6 and CSF2RA blockade but decreased in the presence of GM-CSF and CSF2RB blockade. FAS is downstream of the CSF2 receptor pathway (see, e.g., Takesono et al., Journal of Cell Science 115:3039-3048 (2002)) and its expression is slightly decreased in the presence of GM-CSF blockade with irradiated Nalm6 but not CSF2RA or CSF2RB blockade.

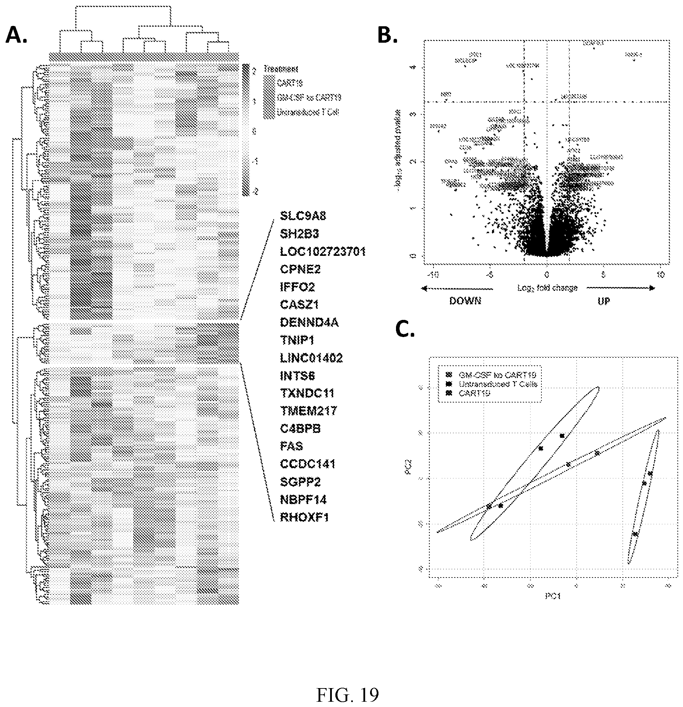

Figures 19A-19C show transcriptome differences between GM-CSF^{k/o} CART19 and CART19 on Day 8 of CART production. Figure 19A shows 236 genes that were significantly differentially expressed (Benjamini-Hochberg adjusted p-value <0.05). Figure 19B shows genes that were significantly downregulated in GM-CSF^{k/o} CART19 versus CART19. A volcano plot shows an increase in significantly downregulated genes between GM-CSF^{k/o} CART19 and CART19. Figure 19C shows that GM-CSF knockout of CART19 normalized gene expression.

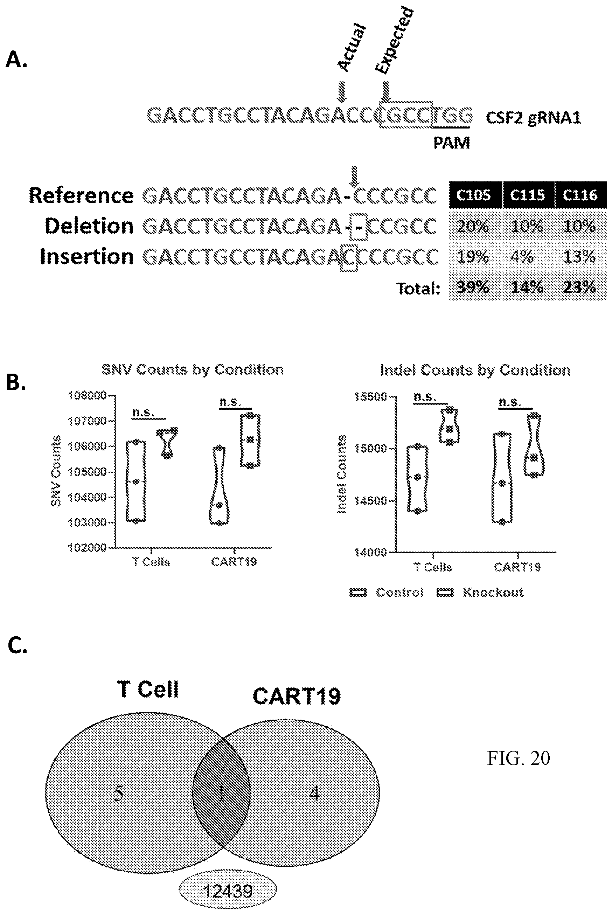

Figures 20A-20C show an exemplary method for precise CSF2 gene-specific editing by CRISPR-Cas9. Figure 20A shows a CSF2 CRISPR gRNA 1 (SEQ ID NO:7) expected cut site (3 bp upstream of PAM) versus actual cut site (6 bp upstream of PAM) (top panel). A reference sequence (SEQ ID NO:1), a deletion schema (SEQ ID NO:8) and an insertion schema (SEQ ID NO:9), and the frequency of deletion and insertion at base 132074828 of chromosome 5 in each biological replicate of CART19 is also shown (bottom panel). Figure 20B shows single nucleotide variant (SNV) counts and insertion/deletion (indel) counts in the CRISPR knockout conditions compared to their controls (T Cells and CART19 cells). Figure 20C shows a representation of the total variants found in CRISPR-edited cells versus their respective control (CART19 or T Cells). The single SNP in the intersection is the deletion in the CSF2 gene (Figure 20A, bottom panel).

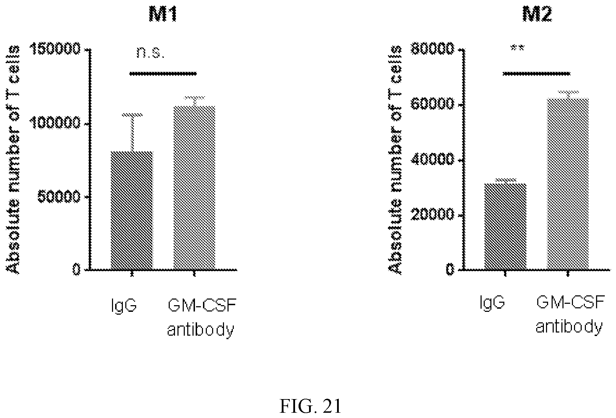

Figure 21 contains graphs showing that GM-CSF blockade in the presence of M2 macrophages significantly enhances CART19 expansion upon CD19 stimulation compared to treatment with isotype control. ** p<0.005.

### DETAILED DESCRIPTION

The present invention provides a method for making a chimeric antigen receptor T cell having a reduced expression level of one or more cytokine GM-CSF polypeptides, said method comprising:
introducing a nucleic acid construct into a T cell ex vivo, wherein the nucleic acid construct comprises: a) a nucleic acid encoding a guide RNA, wherein the guide RNA is complementary to a GM-CSF messenger RNA, b) a nucleic acid encoding a Cas nuclease, and c) a nucleic acid encoding the chimeric antigen receptor,
wherein the nucleic acid encoding the chimeric antigen receptor comprises the nucleic acid sequence set forth in SEQ ID NO: 2 and wherein the chimeric antigen receptor targets CD19 tumor-associated antigen.

In some cases, a T cell (e.g., CART) can be engineered to knock out (KO) a nucleic acid encoding a GM-CSF polypeptide to reduce GM-CSF polypeptide expression in that T cell (*e.g.,* as compared to a T cell that is not engineered to KO a nucleic acid encoding a GM-CSF polypeptide). A T cell that is engineered to KO a nucleic acid encoding a GM-CSF polypeptide can also be referred to herein as a GM-CSF KO T cell. In some cases, the CARTs can be used to modulate myeloid cells. In some cases, the CARTs can be used to deplete myeloid cells. In some cases, the CARTs can be used to enhance T cell (e.g., CARTs) efficacy.

A T cell (e.g., a CART) made by the method of the present invention can be designed to have a reduced expression level of a GM-CSF polypeptide, and optionally also an interleukin 6 (IL-6) polypeptide, a G-CSF, a interferon gamma (IFN-g) polypeptide, an IL-1B polypeptide, an IL-10 polypeptide, a monocyte chemoattractant protein 1 (MCP-1) polypeptide, a monokine induced by gamma (MIG) polypeptide, a macrophage inflammatory protein (MIP) polypeptide (e.g., a MIP-1β polypeptide), a tumor necrosis factor alpha (TNF-a) polypeptide, an IL-2 polypeptide, a perforin polypeptide, or any combination thereof. For example, a T cell can be designed to have a reduced expression level of both GM-CSF and IL-6 polypeptides.

The term "reduced level" as used herein with respect to an expression level of a cytokine (e.g., GM-CSF) refers to any level that is lower than a reference expression level of that cytokine (e.g., GM-CSF). The term "reference level" as used herein with respect to a cytokine (e.g., GM-CSF) refers to the level of that cytokine (e.g., GM-CSF) typically observed in a sample (e.g., a control sample) from one or more mammals (e.g., humans) not engineered to have a reduced expression level of that cytokine (e.g., GM-CSF polypeptides) as described herein. Control samples can include T cells that are wild-type T cells (e.g., T cells that are not GM-CSF KO T cells). In some cases, a reduced expression level of a cytokine polypeptide (e.g., a GM-CSF polypeptide) can be an undetectable level of that cytokine (e.g., GM-CSF). In some cases, a reduced expression level of GM-CSF polypeptides can be an eliminated level of GM-CSF.

In some cases, a T cell having (*e*.*g*., engineered to have) a reduced expression level of one or more cytokine polypeptides such as a GM-CSF KO T cell can maintain normal T cell functions such as T cell degranulation and release of cytokines (*e*.*g*., as compared to a CART that is not engineered to have a reduced expression level of that cytokine (e.g., GM-CSF polypeptides) as described herein).

In some cases, a T cell having (*e*.*g*., engineered to have) a reduced level of GM-CSF polypeptides (*e.g.,* a GM-CSF KO T cell) can have enhanced CART function such as antitumor activity, proliferation, cell killing, cytokine production, exhaustion susceptibility, antigen specific effector functions, persistence, and differentiation (*e*.*g*., as compared to a CART that is not engineered to have a reduced level of GM-CSF polypeptides as described herein).

In some cases, a T cell having (*e*.*g*., engineered to have) a reduced level of GM-CSF polypeptides (*e.g.,* a GM-CSF KO T cell) can have enhanced T cell expansion (*e*.*g*., as compared to a CART that is not engineered to have a reduced level of GM-CSF polypeptides as described herein).

A T cell having (*e*.*g*., engineered to have) a reduced expression level of one or more cytokines (e.g., a GM-CSF polypeptide) such as a GM-CSF KO T cell can be any appropriate T cell. A T cell can be a naive T cell. Examples of T cells that can be designed to have a reduced expression level of one or more cytokines as described herein include cytotoxic T cells (*e*.*g*., CD4+ CTLs and/or CD8+ CTLs). For example, a T cell that can be engineered to have a reduced level of GM-CSF polypeptides as described herein can be a CART. In some cases, one or more T cells can be obtained from a mammal (*e*.*g*., a mammal having cancer). For example, T cells can be obtained from a mammal to be treated as described herein.

A T cell having (*e*.*g*., engineered to have) a reduced expression level of one or more cytokine polypeptides (*e*.*g*., a GM-CSF polypeptide) such as a GM-CSF KO T cell can be generated using any appropriate method. In some cases, a T cell (*e*.*g*., CART) can be engineered to KO a nucleic acid encoding a GM-CSF polypeptide to reduce GM-CSF polypeptide expression in that T cell.

In some cases, when a T cell (*e*.*g*., CART) is engineered to KO a nucleic acid encoding a cytokine (e.g., a GM-CSF polypeptide) to reduce expression of that cytokine polypeptide in that T cell, any appropriate method can be used to KO a nucleic acid encoding that cytokine. Examples of techniques that can be used to knock out a nucleic acid sequence encoding a cytokine polypeptide (e.g., a GM-CSF polypeptide) include gene editing, homologous recombination, non-homologous end joining, and microhomology end joining. For example, gene editing (*e*.*g*., with engineered nucleases) can be used to KO a nucleic acid encoding a GM-CSF polypeptide. Nucleases useful for genome editing include CRISPR-associated (Cas) nucleases, zinc finger nucleases (ZFNs), transcription activator-like effector (TALE) nucleases, and homing endonucleases (HE; also referred to as meganucleases).

In some cases, a clustered regularly interspaced short palindromic repeat (CRISPR) / Cas system can be used (*e*.*g*., can be introduced into one or more T cells) to KO a nucleic acid encoding cytokine polypeptide (e.g., a GM-CSF polypeptide) (see, *e.g.,* Figure 1 and Example 1). A CRISPR/Cas system used to KO a nucleic acid encoding a cytokine polypeptide (e.g., a GM-CSF polypeptide) can include any appropriate guide RNA (gRNA). In some cases, a gRNA can be complementary to a nucleic acid encoding a GM-CSF polypeptide (*e.g.,* a GM-CSF mRNA). Examples of gRNAs that are specific to a nucleic acid encoding a GM-CSF polypeptide include GACCTGCCTACAGACCCGCC (SEQ ID NO:1), GCAGTGCTGCTTGTAGTGGC (SEQ ID NO: 10), TCAGGAGACGCCGGGCCTCC (SEQ ID NO:3), CAGCAGCAGTGTCTCTACTC (SEQ ID NO:4), CTCAGAAATGTTTGACCTCC (SEQ ID NO:5), and GGCCGGTCTCACTCCTGGAC (SEQ ID NO:6). In some cases, a gRNA component of a CRISPR/Cas system designed to KO a nucleic acid encoding a GM-CSF polypeptide can include the nucleic acid sequence set forth in SEQ ID NO:1.

A CRISPR/Cas system used to KO a nucleic acid encoding a cytokine polypeptide (e.g., a GM-CSF polypeptide) can include any appropriate Cas nuclease. Examples of Cas nucleases include Cas1, Cas2, Cas3, Cas9, Cas10, and Cpf1. In some cases, a Cas component of a CRISPR/Cas system designed to KO a nucleic acid encoding a cytokine polypeptide (e.g., a GM-CSF polypeptide) can be a Cas9 nuclease. For example, the Cas9 nuclease of a CRISPR/Cas9 system described herein can be a lentiCRISPRv2 (see, e.g., Shalem et al., 2014 Science 343:84-87; and Sanjana et al., 2014 Nature methods 11: 783-784).

Components of a CRISPR/Cas system (*e.g.,* a gRNA and a Cas nuclease) used to KO a nucleic acid encoding a cytokine polypeptide (e.g., a GM-CSF polypeptide) can be introduced into one or more T cells (*e.g.,* CARTs) in any appropriate format. In some cases, a component of a CRISPR/Cas system can be introduced into one or more T cells as a nucleic acid encoding a gRNA and/or a nucleic acid encoding a Cas nuclease. For example, a nucleic acid encoding at least one gRNA (*e.g.,* a gRNA sequence specific to a nucleic acid encoding a GM-CSF polypeptide) and a nucleic acid at least one Cas nuclease (*e.g.,* a Cas9 nuclease) can be introduced into one or more T cells. In some cases, a component of a CRISPR/Cas system can be introduced into one or more T cells as a gRNA and/or as a Cas nuclease. For example, at least one gRNA (*e.g.,* a gRNA sequence specific to a nucleic acid encoding a GM-CSF polypeptide) and at least one Cas nuclease (*e.g.,* a Cas9 nuclease) can be introduced into one or more T cells.

The components of a CRISPR/Cas system (*e.g.,* a gRNA and a Cas nuclease) are introduced into one or more T cells as a nucleic acid construct encoding the components (*e*.*g*., nucleic acid encoding a gRNA and nucleic acid encoding a Cas nuclease), *e.g.,* an expression construct. A nucleic acid encoding at least one gRNA and a nucleic acid encoding at least one Cas nuclease can be on separate nucleic acid constructs or on the same nucleic acid construct. In some cases, a nucleic acid encoding at least one gRNA and a nucleic acid encoding at least one Cas nuclease can be on a single nucleic acid construct. A nucleic acid construct can be any appropriate type of nucleic acid construct. Examples of nucleic acid constructs that can be used to express at least one gRNA and/or at least one Cas nuclease include expression plasmids and viral vectors (*e*.*g*., lentiviral vectors). In cases where a nucleic acid encoding at least one gRNA and a nucleic acid encoding at least one Cas nuclease are on separate nucleic acid constructs, the nucleic acid constructs can be the same type of construct or different types of constructs. In some cases, a nucleic acid encoding at least one gRNA sequence specific to a nucleic acid encoding a cytokine polypeptide (e.g., a GM-CSF polypeptide) and a nucleic acid encoding at least one Cas nuclease can be on a single lentiviral vector. For example, a lentiviral vector encoding at least one gRNA sequence specific to a nucleic acid encoding a cytokine polypeptide (e.g., GM-CSF polypeptide), encoding at least one gRNA including the sequence set forth in SEQ ID NO:1, and encoding at least one Cas9 nuclease can be used in *ex vivo* engineering of T cells to have a reduced expression level of that cytokine (e.g., a GM-CSF polypeptide).

In some cases, components of a CRISPR/Cas system (*e.g.,* a gRNA and a Cas nuclease) can be introduced directly into one or more T cells (*e.g.,* as a gRNA and/or as Cas nuclease). A gRNA and a Cas nuclease can be introduced into the one or more T cells separately or together. In cases where a gRNA and a Cas nuclease are introduced into the one or more T cells together, the gRNA and the Cas nuclease can be in a complex. When a gRNA and a Cas nuclease are in a complex, the gRNA and the Cas nuclease can be covalently or non-covalently attached. In some cases, a complex including a gRNA and a Cas nuclease also can include one or more additional components. Examples of complexes that can include components of a CRISPR/Cas system (*e.g.,* a gRNA and a Cas nuclease) include ribonucleoproteins (RNPs) and effector complexes (e.g., containing a CRISPR RNAs (crRNAs) a Cas nuclease). For example, at least one gRNA and at least one Cas nuclease can be included in a RNP. In some cases, a RNP can include gRNAs and Cas nucleases at a ratio of about 1:1 to about 10:1 (e.g., about 1:1 to about 10:1, about 2:1 to about 10:1, about 3:1 to about 10:1, about 5:1 to about 10:1, about 8:1 to about 10:1, about 1:1 to about 9:1, about 1:1 to about 7:1, about 1:1 to about 5:1, about 1:1 to about 4:1, about 1:1 to about 3:1, about 1:1 to about 2:1, about 2:1 to about 8:1, about 3:1 to about 6:1, about 4:1 to about 5:1, or about 5:1 to about 7:1). For example, a RNP can include gRNAs and Cas nucleases at about a 1:1 ratio. For example, a RNP can include gRNAs and Cas nucleases at about a 2:1 ratio. In some cases, a RNP including at least one gRNA sequence specific to a nucleic acid encoding a GM-CSF polypeptide (*e*.*g*., encoding at least one gRNA including the sequence set forth in SEQ ID NO:1) and at least one Cas9 nuclease can be used in *ex vivo* engineering of T cells to have a reduced level of GM-CSF polypeptides.

Components of a CRISPR/Cas system (*e*.*g*., a gRNA and a Cas nuclease) used to KO a nucleic acid encoding a GM-CSF polypeptide can be introduced into one or more T cells (*e*.*g*., CARTs) using any appropriate method. A method of introducing components of a CRISPR/Cas system into a T cell can be a physical method. A method of introducing components of a CRISPR/Cas system into a T cell can be a chemical method. A method of introducing components of a CRISPR/Cas system into a T cell can be a particle-based method. Examples of methods that can be used to introduce components of a CRISPR/Cas system into one or more T cells include electroporation, transfection (*e*.*g*., lipofection), transduction (*e*.*g*., viral vector mediated transduction), microinjection, and nucleofection. In some cases, when components of a CRISPR/Cas system are introduced into one or more T cells as nucleic acid encoding the components, the nucleic acid encoding the components can be transduced into the one or more T cells. For example, a lentiviral vector encoding at least one gRNA sequence specific to a nucleic acid encoding a GM-CSF polypeptide (*e*.*g*., encoding at least one gRNA including the sequence set forth in SEQ ID NO:1) and at least one Cas9 nuclease can be transduced into T cells (*e.g., ex vivo* T cells). In some cases, when components of a CRISPR/Cas system are introduced directly into one or more T cells, the components can be electroporated into the one or more T cells. For example, a RNP including at least one gRNA sequence specific to a nucleic acid encoding a GM-CSF polypeptide (*e*.*g*., encoding at least one gRNA including the sequence set forth in SEQ ID NO:1) and at least one Cas9 nuclease can be electroporated into T cells (*e.g., ex vivo* T cells). In some cases, components of a CRISPR/Cas system can be introduced *ex vivo* into one or more T cells. For example, *ex vivo* engineering of T cells have a reduced level of GM-CSF polypeptides can include transducing isolated T cells with a lentiviral vector encoding components of a CRISPR/Cas system. For example, *ex vivo* engineering of T cells having reduced levels of GM-CSF polypeptides can include electroporating isolated T cells with a complex including components of a CRISPR/Cas system. In cases where T cells are engineered *ex vivo* to have a reduced level of GM-CSF polypeptides, the T cells can be obtained from any appropriate source (*e.g.,* a mammal such as the mammal to be treated or a donor mammal, or a cell line).

In some cases, a T cell (*e.g.,* a CART) can be treated with one or more inhibitors of GM-CSF polypeptide expression or GM-CSF polypeptide activity to reduce GM-CSF polypeptide expression in that T cell (*e.g.,* as compared to a T cell that was not treated with one or more inhibitors of GM-CSF polypeptide expression or GM-CSF polypeptide activity). An inhibitor of GM-CSF polypeptide expression or GM-CSF polypeptide activity can be any appropriate inhibitor. Example of inhibitors of GM-CSF polypeptide expression or GM-CSF polypeptide activity include nucleic acid molecules designed to induce RNA interference (*e.g.,* a siRNA molecule or a shRNA molecule), antisense molecules, miRNAs, receptor blockade, and antibodies (*e*.*g*., antagonistic antibodies and neutralizing antibodies).

A T cell having (*e*.*g*., engineered to have) a reduced expression level of one or more cytokines (*e*.*g*., a GM-CSF KO T cell) can express (*e*.*g*., can be engineered to express) any appropriate antigen receptor. In some cases, an antigen receptor can be a heterologous antigen receptor. In some cases, an antigen receptor can be a CAR. In some cases, an antigen receptor can be a tumor antigen (*e*.*g*., tumor-specific antigen) receptor. For example, a T cell can be engineered to express a tumor-specific antigen receptor that targets a tumor-specific antigen (*e.g.,* a cell surface tumor-specific antigen) expressed by a cancer cell in a mammal having cancer. Examples of antigens that can be recognized by an antigen receptor expressed in a T cell having reduced expression of a cytokine polypeptide (e.g., a GM-CSF polypeptide) as described herein include cluster of differentiation 19 (CD19), mucin 1 (MUC-1), human epidermal growth factor receptor 2 (HER-2), estrogen receptor (ER), epidermal growth factor receptor (EGFR), alphafetoprotein (AFP), carcinoembryonic antigen (CEA), CA-125, epithelial tumor antigen (ETA), melanoma-associated antigen (MAGE), CD33, CD123, CLL-1, E-Cadherin, folate receptor alpha, folate receptor beta, IL13R, EGFRviii, CD22, CD20, kappa light chain, lambda light chain, desmopressin, CD44v, CD45, CD30, CD5, CD7, CD2, CD38, BCMA, CD138, FAP, CS-1, and C-met. For example, a T cell having a reduced level of GM-CSF polypeptides can be designed to express an antigen receptor targeting CD19. An exemplary nucleic acid sequence encoding a CAR targeting CD19 (CAR19) is shown in Figure 5.

Any appropriate method can be used to express an antigen receptor on a T cell having (*e*.*g*., engineered to have) a reduced expression level of one or more cytokine polypeptides (*e.g.,* a GM-CSF KO T cell). For example, a nucleic acid encoding an antigen receptor can be introduced into one or more T cells. In some cases, viral transduction can be used to introduce a nucleic acid encoding an antigen receptor into a non-dividing a cell. A nucleic acid encoding an antigen receptor can be introduced in a T cell using any appropriate method. In some cases, a nucleic acid encoding an antigen receptor can be introduced into a T cell by transduction (*e*.*g*., viral transduction using a retroviral vector such as a lentiviral vector) or transfection. In some cases, a nucleic acid encoding an antigen receptor can be introduced *ex vivo* into one or more T cells. For example, *ex vivo* engineering of T cells expressing an antigen receptor can include transducing isolated T cells with a lentiviral vector encoding an antigen receptor. In cases where T cells are engineered *ex vivo* to express an antigen receptor, the T cells can be obtained from any appropriate source (*e*.*g*., a mammal such as the mammal to be treated or a donor mammal, or a cell line).

In some cases, when a T cell having (*e*.*g*., engineered to have) a reduced expression level of one or more cytokine polypeptides (*e.g.,* a GM-CSF KO T cell) also expresses (*e*.*g*., is engineered to express) an antigen receptor, that T cell can be engineered to have a reduced expression level of that cytokine and engineered to express an antigen receptor using any appropriate method. In some cases, a T cell can be engineered to have a reduced expression level of a cytokine polypeptide (e.g., a GM-CSF polypeptide) first and engineered to express an antigen receptor second, or vice versa. In some cases, a T cell can be simultaneously engineered to have a reduced expression level of one or more cytokine polypeptides (e.g., a GM-CSF polypeptide) and to express an antigen receptor. For example, one or more nucleic acids used to reduce expression of a cytokine polypeptide such as a GM-CSF polypeptide (e.g., a lentiviral vector encoding at least one gRNA sequence specific to a nucleic acid encoding that cytokine and at least one Cas9 nuclease or a nucleic acid encoding at least one oligonucleotide that is complementary to that cytokine's mRNA) and one or more nucleic acids encoding an antigen receptor (*e*.*g*., a CAR) can be simultaneously introduced into one or more T cells. One or more nucleic acids used to reduce expression of a cytokine polypeptide (e.g., a GM-CSF polypeptide) and one or more nucleic acids encoding an antigen receptor can be introduced into one or more T cells on separate nucleic acid constructs or on a single nucleic acid construct. In some cases, one or more nucleic acids used to reduce expression of a cytokine polypeptide (e.g., a GM-CSF polypeptide) and one or more nucleic acids encoding an antigen receptor can be introduced into one or more T cells on a single nucleic acid construct. In some cases, one or more nucleic acids used to reduce expression of a cytokine polypeptide (e.g., a GM-CSF polypeptide) and one or more nucleic acids encoding an antigen receptor can be introduced *ex vivo* into one or more T cells. In cases where T cells are engineered *ex vivo* to have a reduced expression levels of one or more cytokine polypeptides (e.g., a GM-CSF polypeptide) and to express an antigen receptor, the T cells can be obtained from any appropriate source (*e.g.,* a mammal such as the mammal to be treated or a donor mammal, or a cell line).

In some cases, a T cell having (*e*.*g*., engineered to have) a reduced expression level of one or more cytokine polypeptides (*e.g.,* a GM-CSF KO T cell) can be stimulated. A T cell can be stimulated at the same time as being engineered to have a reduced level of one or more cytokine polypeptides or independently of being engineered to have a reduced level of one or more cytokine polypeptides. For example, one or more T cells having a reduced level of GM-CSF polypeptides used in an adoptive cell therapy can be stimulated first, and can be engineered to have a reduced expression level of GM-CSF polypeptides second, or vice versa. In some cases, one or more T cells having a reduced expression level of a cytokine polypeptide (e.g., a GM-CSF polypeptide) used in an adoptive cell therapy can be stimulated first, and can be engineered to have a reduced level of that cytokine polypeptide second. A T cell can be stimulated using any appropriate method. For example, a T cell can be stimulated by contacting the T cell with one or more CD polypeptides. Examples of CD polypeptides that can be used to stimulate a T cell include CD3, CD28, inducible T cell co-stimulator (ICOS), CD137, CD2, OX40, and CD27. In some cases, a T cell can be stimulated with CD3 and CD28 prior to introducing components of a CRISPR/Cas system (*e.g.,* a gRNA and/or a Cas nuclease) to the T cell to KO a nucleic acid encoding one or more cytokine polypeptides (e.g., a GM-CSF polypeptide).

The present invention also provides a chimeric antigen receptor T cell having a reduced level of granulocyte-macrophage colony-stimulating factor (GM-CSF) polypeptides for use in the treatment of cancer, wherein the chimeric antigen receptor T cell comprises a chimeric antigen receptor encoded by the nucleic acid sequence set forth in SEQ ID NO: 2 and wherein the chimeric antigen receptor targets CD19 tumor-associated antigen.

For example, one or more T cells having (e.g., engineered to have) a reduced expression level of a cytokine polypeptide (*e.g.,* a GM-CSF KO T cells) can be administered (*e.g.,* in an adoptive cell therapy such as a CART therapy) to a mammal (*e.g.,* a human) having cancer. In some cases, the chimeric antigen receptor T cell used in treating a mammal having cancer as described herein can reduce the number of cancer cells (*e.g.,* cancer cells expressing a tumor antigen) within the mammal. In some cases, the chimeric antigen receptor T cell used in treating a mammal having cancer as described herein can reduce the size of one or more tumors (*e*.*g*., tumors expressing a tumor antigen) within a mammal.

In some cases, administering T cells having (*e*.*g*., engineered to have) a reduced expression level of a cytokine polypeptide (*e.g.,* a GM-CSF KO T cell) to a mammal does not result in CRS. For example, administering T cells having a reduced level of GM-CSF polypeptides to a mammal does not result in release of cytokines associated with CRS (*e.g.,* CRS critical cytokines). Examples of cytokines associated with CRS include IL-6, G-CSF, IFN-g, IL-1B, IL-10, MCP-1, MIG, MIP, MIP 1b, TNF-a, IL-2, and perforin.

In some cases, administering T cells having (*e*.*g*., engineered to have) a reduced expression level of a cytokine polypeptide (*e.g.,* a GM-CSF KO T cell) to a mammal does not result in neurotoxicity. For example, administering T cells having a reduced level of GM-CSF polypeptides to a mammal does not result in differentiation and/or activation of white blood cells, the differentiation and/or activation of which, is associated with neurotoxicity. Examples of white blood cells, the differentiation and/or activation of which, is associated with neurotoxicity include monocytes, macrophages, T-cells, dendritic cells, microglia, astrocytes, and neutrophils.

Any appropriate mammal (*e*.*g*., a human) having a cancer can be treated as described herein. Examples of mammals that can be treated as described herein include humans, primates (such as monkeys), dogs, cats, horses, cows, pigs, sheep, mice, and rats. For example, a human having a cancer can be treated with one or more T cells having (*e*.*g*., engineered to have) a reduced expression level of a GM-CSF polypeptide in, for example, an adoptive T cell therapy such as a CART cell therapy as described herein.

When treating a mammal (*e*.*g*., a human) having a cancer as described herein, the cancer can be any appropriate cancer. In some cases, the cancer can be a solid tumor. In some cases, the cancer can be a hematological cancer. In some cases, the cancer can be a primary cancer. In some cases, the cancer can be a metastatic cancer. In some cases, the cancer can be a refractory cancer. In some cases, the cancer can be a relapsed cancer. In some cases, the cancer can express a tumor-associated antigen (*e.g.,* an antigenic substance produced by a cancer cell). Examples of cancers that can be treated as described herein include B cell cancers (e.g., diffuse large B cell lymphoma (DLBCL) and B cell leukemias), acute lymphoblastic leukemia (ALL), chronic lymphocytic leukemia (CLL), follicular lymphoma, mantle cell lymphoma, non-Hodgkin lymphoma, Hodgkin lymphoma, acute myeloid leukemia (AML), multiple myeloma, head and neck cancers, sarcomas, breast cancer, gastrointestinal malignancies, bladder cancers, urothelial cancers, kidney cancers, lung cancers, prostate cancers, ovarian cancers, cervical cancers, genital cancers (e.g., male genital cancers and female genital cancers), and bone cancers. For example, one or more T cells having (*e.g.,* engineered to have) a reduced level of GM-CSF polypeptides (*e.g.,* a GM-CSF KO T cells) can be used in treating a mammal having DLBCL. For example, one or more T cells having (*e.g.,* engineered to have) a reduced level of GM-CSF polypeptides (*e.g.,* a GM-CSF KO T cells) can be used in treating a mammal having ALL.

Any appropriate method can be used to identify a mammal having cancer. For example, imaging techniques and biopsy techniques can be used to identify mammals (*e*.*g*., humans) having cancer.

Once identified as having a cancer (*e.g.,* DLBCL or ALL), a mammal can be administered one or more T cells having (*e*.*g*., engineered to have) a reduced expression level of GM-CSF polypeptide (*e.g.,* a GM-CSF KO T cells) described herein.

For example, one or more T cells having (*e*.*g*., engineered to have) a reduced expression level of GM-CSF polypeptide (*e.g.,* a GM-CSF KO T cells) can be used in an adoptive T cell therapy (*e.g.,* a CART cell therapy) to treat a mammal having a cancer. For example, one or more T cells having a reduced level of GM-CSF polypeptides can be used in an adoptive T cell therapy (*e.g.,* a CART cell therapy) targeting any appropriate antigen within a mammal (*e*.*g*., a mammal having cancer). In some cases, an antigen can be a tumor-associated antigen (*e.g.,* an antigenic substance produced by a cancer cell). Examples of tumor-associated antigens that can be targeted by an adoptive T cell therapy include CD19 (associated with DLBCL, ALL, and CLL), AFP (associated with germ cell tumors and/or hepatocellular carcinoma), CEA (associated with bowel cancer, lung cancer, and/or breast cancer), CA-125 (associated with ovarian cancer), MUC-1 (associated with breast cancer), ETA (associated with breast cancer), MAGE (associated with malignant melanoma), CD33 (associated with AML), CD123 (associated with AML), CLL-1 (associated with AML), E-Cadherin (associated with epithelial tumors), folate receptor alpha (associated with ovarian cancers), folate receptor feta (associated with ovarian cancers and AML), IL13R (associated with brain cancers), EGFRviii (associated with brain cancers), CD22 (associated with B cell cancers), CD20 (associated with B cell cancers), kappa light chain (associated with B cell cancers), lambda light chain (associated with B cell cancers), CD44v (associated with AML), CD45 (associated with hematological cancers), CD30 (associated with Hodgkin lymphomas and T cell lymphomas), CD5 (associated with T cell lymphomas), CD7 (associated with T cell lymphomas), CD2 (associated with T cell lymphomas), CD38 (associated with multiple myelomas and AML), BCMA (associated with multiple myelomas), CD138 (associated with multiple myelomas and AML), FAP (associated with solid tumors), CS-1 (associated with multiple myeloma), and c-Met (associated with breast cancer). For example, one or more T cells having a reduced level of GM-CSF polypeptides can be used in CART cell therapy targeting CD19 (*e*.*g*., CART19 cell therapy) to treat cancer.

In some cases, one or more T cells having (*e*.*g*., engineered to have) a reduced expression level of GM-CSF polypeptide (*e*.*g*., a GM-CSF KO T cells) can be used in an adoptive T cell therapy (*e*.*g*., a CART cell therapy) to treat a mammal having a disease or disorder other than cancer. For example, one or more T cells having a reduced level of GM-CSF polypeptides can be used in an adoptive T cell therapy (*e*.*g*., a CART cell therapy) targeting any appropriate disease-associated antigen (*e*.*g*., an antigenic substance produced by cell affected by a particular disease) within a mammal. Examples of disease-associated antigens that can be targeted by an adoptive T cell therapy include desmopressin (associated with auto immune skin diseases).

In some cases, one or more T cells having (*e*.*g*., engineered to have) a reduced expression level of GM-CSF polypeptide (*e*.*g*., a GM-CSF KO T cells) used in an adoptive T cell therapy (*e*.*g*., a CART cell therapy) can be administered to a mammal having a cancer as a combination therapy with one or more additional agents used to treat a cancer. For example, one or more T cells having a reduced level of GM-CSF polypeptides used in an adoptive cell therapy can be administered to a mammal in combination with one or more anti-cancer treatments (*e*.*g*., surgery, radiation therapy, chemotherapy (*e*.*g*., alkylating agents such as busulfan), targeted therapies (*e*.*g*., GM-CSF inhibiting agents such as lenzilumab), hormonal therapy, angiogenesis inhibitors, immunosuppressants (*e*.*g*., interleukin-6 inhibiting agents such as tocilizumab)) and/or one or more CRS treatments (*e*.*g*., ruxolitinib and ibrutinib). In cases where one or more T cells having a reduced level of GM-CSF polypeptides used in an adoptive cell therapy are used with additional agents in treating a cancer, the one or more additional agents can be administered at the same time or independently. In some cases, one or more T cells having a reduced level of GM-CSF polypeptides used in an adoptive cell therapy can be administered first, and the one or more additional agents administered second, or vice versa.

The invention will be further described in the following examples, which do not limit the scope of the invention described in the claims.

### EXAMPLES

### Example 1: Generation of cytokine to deficient CART cells to increase therapeutic index of CART cell therapy

This example describes the development of GM-CSF knocked out (GM-CSF KO) CART19 cells, and shows that the resulting GM-CSF KO CART19 cells function normally and have enhanced expansion.

### Experimental design:

CAR19 in B cell leukemia xenografts were used. These plasmids were used for packaging and lentivirus production as described herein. As a mouse model, two models were employed:
1. Xenograft models: NSG mice were subcutaneously engrafted with the CD19 positive, luciferase positive cell line NALM6. Engraftment was confirmed by bioluminescence imaging. Mice were treated with human PBMCs intravenously and intra-tumor injection of lentivirus particles. Generation of CART cells is measured by flow cytometry. Trafficking of CARTs to tumor sites is assessed and anti-tumor response is measured by bioluminescence imaging as a measure of disease burden.
2. Humanized Immune System (HIS) mice from the Jackson Laboratory: These mice were injected with fetal CD34+ cells as neonates and therefore develop human hematopoiesis. We will engraft these mice with the CD19+ cell line NALM6, as previously used. Similarly, we will generate CART19 *in vivo* through the intratumoral injection of lentivirus particles. Then will measure the activity of CART19 cells in eradication of NALM6 and compare that to ex vivo generated lenti-virally transduced CART19 cells (currently used in the clinic).

### Materials and Methods:

### Generation of CAR plasmid:

The anti-CD19 clone FMC63 was do novo synthesized into a CAR backbone using 41BB and CD3 zeta and then cloned into a third generation lentivirus backbone.

To generate the control CART19 cells, normal donor T cells were negatively selected using pan T cell kit and expanded ex vivo using anti-CD3/CD28 Dynabeads (Invitrogen, added on the first day of culture). T cells were transduced with lentiviral supernatant one day following stimulation at a multiplicity of infection (MOI) of 3. The anti-CD3/CD28 Dynabeads were removed on day 6 and T cells were grown in T cell media (X-vivo 15 media, human serum 5%, penicillin, streptomycin and glutamine) for up to 15 days and then cryopreserved for future experiments. Prior to all experiments, T cells were thawed and rested overnight at 37°C.

### Generation of GM-CSF knock out CART cells:

GM-CSF knockout CART cells were generated with a CRISR-Cas9 system, using two methodologies:
1. gRNA was generated and cloned into a lentivirus vector that encodes Cas9 and the gRNA. During T cell expansion, T cells were transduced with this lentivirus on Day 1, on the same day and simultaneously with CAR19 lentivirus particles. Cells were expanded for a period of 8 days and then T cell were harvested, DNA isolated and sequenced to assess the efficiency of knockout. These cells were cryopreserved and used for future *in vitro* or *in vivo* experiments. A nucleic acid sequence encoding is shown in Figure 5.
2. mRNA was generated from the gRNA and used it to knock out GM-CSF. To do so, gRNA was mixed with RNP at 1:1 ratio and then T cells were electroporated on Day 3 post stimulation with CD3/CD28 beads. Cells were expanded for a period of 8 days and then T cell were harvested, DNA isolated and sequenced to assess the efficiency of knockout. These cells were cryopreserved and used for future *in vitro* or *in vivo* experiments

### Cells

The NALM6 cell line was obtained from the ATCC and maintained in R10 media (RPMI media, 10% fetal calf serum, penicillin, and streptomycin). NALM6-cells transduced with luciferase-GFP cells under the control of the EF1α promoter were used in some experiments as indicated. De-identified primary human ALL specimens were obtained from the Mayo Clinic Biobank. All samples were obtained after informed, written consent. For all functional studies, cells were thawed at least 12 hours before analysis and rested overnight at 37°C.

### Flow cytometry analysis

Anti-human antibodies were purchased from BioLegend, eBioscience, or BD Biosciences. Cells were isolated from *in vitro* culture or from animals, washed once in PBS supplemented with 2% fetal calf serum, and stained at 4°C after blockade of Fc receptors. For cell number quantitation, Countbright beads (Invitrogen) were used according to the manufacturer's instructions (Invitrogen). In all analyses, the population of interest was gated based on forward vs. side scatter characteristics followed by singlet gating, and live cells were gated using Live Dead Aqua (Invitrogen). Surface expression of anti-CD19 CAR was detected by staining with an Alexa Fluor 647-conjugated goat anti-mouse F(ab')2 antibody from Jackson Immunoresearch.

### T cell function assays:

### T cell degranulation and intracellular cytokine assays:

Briefly, T cells were incubated with target cells at a 1:5 ratio. After staining for CAR expression; CD107a, CD28, CD49d and monensin were added at the time of incubation. After 4 hours, cells were harvested and stained for CAR expression, CD3 and Live Dead staining (Invitrogen). Cells were fixed and permeabilized (FIX & PERM^{®} Cell Fixation & Cell Permeabilization Kit, Life technologies) and intracellular cytokine staining was then performed.

### Proliferation assays:

T cells were washed and resuspended at 1×10⁷/ml in 100 µl of PBS and labeled with 100 µl of CFSE 2.5µM (Life Technologies) for 5 minutes at 37°C. The reaction was then quenched with cold R10, and the cells were washed three times. Targets were irradiated at a dose of 100 Gy. T cells were incubated at a 1:1 ratio with irradiated target cells for 120 hours. Cells were then harvested, stained for CD3, CAR and Live Dead aqua (Invitrogen), and Countbright beads (Invitrogen) were added prior to flow cytometric analysis

### Cytotoxicity assays:

NALM6-Luc cells or CFSE (Invitrogen) labelled primary ALL samples were used for cytotoxicity assay. In brief, targets were incubated at the indicated ratios with effector T cells for 4, 16, 24, 48, and/or 72 hours. Killing was calculated either by bioluminescence imaging on a Xenogen IVIS-200 Spectrum camera or by flow cytometry. For the latter, cells were harvested; Countbright beads and 7-AAD (Invitrogen) were added prior to analysis. Residual live target cells were CFSE+ 7-AAD-.

### Secreted cytokine measurement:

Effector and target cells were incubated at a 1:1 ratio in T cell media for 24 or 72 hours as indicated. Supernatant was harvested and analyzed by 30-plex Luminex array according to the manufacturer's protocol (Invitrogen).

### Results

GM-CSF KO CART cells were generated with a CRISR-Cas9 system. During T cell expansion, T cells were transduced (Day 1) with lentivirus encoding gRNA and Cas9 and lentivirus encoding CAR19. Cells were expanded for a period of 8 days. After 8 days, T cells were harvested, DNA was isolated, and the isolated DNA was sequenced to assess the efficiency of knockout. See, *e.g.,* Figure 1. T cells exhibited a knockout efficiency of 24.1% (Figure 2A), and CAR transduction efficiency was 73% (Figure 2B).

To evaluate cell effector functions of GM-CSF KO CART cells, CART19, GM-CSF KO CART19, UTD, or GM-CSF KO UTD were co-cultured with the CD19 positive cell line NALM6 at a ratio of 1:5. After 4 hours, the cells were harvested, permeabilized, fixed, and stained for cytokines (Figure 3).

To evaluate proliferation of GM-CSF KO CART cells, expansion kinetics were followed after T cells were transduced. GM-CSF KO CART cells expand more robustly than cells transduced with CART19 alone (Figure 4).

These results demonstrate that GM-CSF knockout CARTs can enhance CART cell function and antitumor activity. These results also demonstrate that blockade of GMCSF in combination with CART19 does not impact CART cell effector functions.

### Example 2: GM-CSF depletion during CART therapy reduces cytokine release syndrome and neurotoxicity and may enhance CART cell function

This example investigates depleting granulocyte macrophage colony-stimulating factor (GM-CSF) and myeloid cells as a potential strategy to manage CART cell associated toxicities. It was found that the GM-CSF blockade with a neutralizing antibody does not does not inhibit CART function *in vitro* or *in vivo.* CART cell proliferation was enhanced *in vitro* and CART cells resulted in a more efficient control of leukemia in patient derived xenografts after GM-CSF depletion. Furthermore, in a primary acute lymphoblastic leukemia xenograft model of CRS and NT, GM-CSF blockade resulted in a reduction of myeloid cell and T cell infiltration in the brain, and ameliorated the development of CRS and NT. Finally, GM-CSF knocked out CART cells were generated through CRISPR/cas9 disruption of GM-CSF during CART cell manufacturing. GM-CSF^{k/o} CART cells continued to function normally and had resulted in enhanced anti-tumor activity *in vivo.* These demonstrate that GM-CSF neutralization can abrogate neurotoxicity and CRS, and also can enhance CART cell functions.

### Materials and Methods

### Cells lines and primary cells

NALM6 and MOLM13 were purchased from ATCC, Manassas, VA, USA, transduced with a luciferase-ZsGreen lentivirus (addgene) and sorted to 100% purity. Cell lined were cultured in R10 (RPMI, 10% FCS v/v, 1% pen strep v/v). Primary cells were obtained from the Mayo Clinic biobank for patients with acute leukemia under an institutional review board approved protocol. The use of recombinant DNA in the laboratory was approved by the Institutional Biosafety Committee (IBC)

### Primary T cells and CART cells

Peripheral blood mononuclear cells (PBMC) were isolated from de-identified donor blood apheresis cones using a FICOLL protocol (see, *e.g.,* Dietz et al., 2006 Transfusion 46:2083-2089). T cells were separated with negative selection magnetic beads (Stemcell technologies) and monocytes were positively selected using CD14+ magnetic beads (Stemcell technologies). Primary cells were cultured in X-Vivo 15 media with 5% human serum, penicillin, streptomycin and glutamax. CD19 directed CART cells were generated through the lentiviral transduction of normal donor T cells as described below. Second generation CAR19 constructs were *do novo* synthesized (IDT) and cloned into a third generation lentivirus under the control of EF-1α promotor. The CD19 directed single chain variable fragment was derived from the clone FMC63. A second generation 41BB co-stimulated (FMC63-41BBz) CAR construct was synthesized and used for these experiments. Lentivirus particles were generated through the transient transfection of plasmid into 293T virus producing cells, in the presence of lipofectamine 3000, VSV-G and packaging plasmids. T cells isolated from normal donors were stimulated using CD3/CD28 stimulating beads (StemCell) at 1:3 ratio and then transduced with lentivirus particles 24 hours after stimulation at a multiplicity of infection of 3.0. Magnetic bead removal was performed on Day 6 and CART cells were harvested and cryopreserved on Day 8 for future experiments. CART cells were thawed and rested in T cell medium 12 hours prior to their use in experiments.

### Generation of GM-CSF^{k/}^{o} CART cells:

A guide RNA (gRNA) targeting exon 3 of human GM-CSF was selected via screening gRNAs previously reported to have high efficiency for human GM-CSF.25 This gRNA was ordered in a CAS9 third generation lentivirus construct (lentiCRISPRv2), controlled under a U6 promotor (GenScript, Township, NJ, USA). Lentiviral particles encoding this construct were produced as described above. T cells were dual transduced with CAR19 and GM-CSFgRNA-lentiCRISPRv2 lentiviruses, 24 hours after stimulation with CD3/CD28 beads. CAR-T cell expansion was then continued as described above. To analyze efficiency of targeting GM-CSF, genomic DNA was extracted from the GM-CSF^{k/o} CART19 cells using PureLink Genomic DNA Mini Kit (Invitrogen, Carlsbad, CA, USA). The DNA of interest was PCR amplified using Choice Taq Blue Mastermix (Thomas Scientific, Minneapolis, MN, USA) and gel extracted using QIAquick Gel Extraction Kit (Qiagen, Germantown, MD, USA) to determine editing. PCR amplicons were sent for Eurofins sequencing (Louisville, KY, USA) and allele modification frequency was calculated using TIDE (Tracking of Indels by Decomposition) software available at tide.nki.nl. Figure 15 describes the gRNA sequence, primer sequences, and the schema for generation of GM-CSF^{k/o} CART19 schema.

### GM-CSF neutralizing antibodies and isotype controls

Lenzilumab (Humanigen, Brisbane, CA) is a humanized antibody that neutralizes human GM-CSF. For *in vitro* experiments, lenzilumab or isotype control 10 ug/mL was used. For *in vivo* experiments, 10 mg/kg of lenzilumab or isotype control was injected, and the schedule, route and frequency are indicated in the individual experimental schema. In some experiments, anti-mouse GM-CSF neutralizing antibody (10 mg/kg) was also used, as indicated in the experimental schema.

### T cell functional experiments

Cytokine assays were performed 24 or 72 hours after a co-culture of CART cells with their targets at 1:1 ratio as indicated. Human GM-CSF singleplex (Millipore), 30-plex human multiplex (Millipore), or 30-plex mouse multiplex (Millipore) was performed on supernatant collected from these experiments, as indicated. This was analyzed using flow cytometry bead assay or Luminex, Intracellular cytokine analysis and T cell degranulation assays were performed following incubation of CART cells with targets at 1:5 ratio for 4 hours at 37 °C, in the presence of monensin, hCD49d, and hCD28. After 4 hours, cells were harvested and intracellular staining was performed after surface staining, followed by fixation and permealization (FIX & PERM Cell Fixation & Cell Permeabilization Kit, Life Technologies). For proliferation assays, CFSE (Life Technologies) labeled effector cells (CART19), and irradiated target cells were co cultured at 1:1. In some experiments with CD14+ monocytes was added to the co-culture at 1:1:1 ratio as indicated. Cells were co-cultured for 3-5 days, as indicated in the specific experiment and then cells were harvested and surface staining with anti-hCD3 and live/dead aqua was performed. PMA/ionomycin was used as a positive non-specific stimulant of T cells, at different concentrations as indicated in the specific experiments. For killing assays, the CD19⁺Luciferase⁺ ALL cell line NALM6 or the CD19⁻Luciferase⁺ control MOLM13 cells were incubated at the indicated ratios with effector T cells for 24 or 48 hours as listed in the specific experiment. Killing was calculated by bioluminescence imaging on a Xenogen IVIS-200 Spectrum camera (PerkinElmer, Hopkinton, MA, USA) as a measure of residual live cells. Samples were treated with 1 µl D-luciferin (30ug/mL) per 100 µl sample volume, 10 minutes prior to imaging.

### Multi-parametric flow cytometry

Anti-human antibodies were purchased from Biolegend, eBioscience, or BD Biosciences. Cells were isolated from *in vitro* culture or from peripheral blood of animals (after ACK lysis), washed twice in phosphate-buffered saline supplemented with 2% fetal calf serum and stained at 4 °C. For cell number quantitation, Countbright beads (Invitrogen) were used according to the manufacturer's instructions (Invitrogen). In all analyses, the population of interest was gated based on forward vs side scatter characteristics, followed by singlet gating, and live cells were gated using Live Dead Aqua (Invitrogen). Surface expression of CAR was detected by staining with a goat anti-mouse F(ab')2 antibody. Flow cytometry was performed on a four-laser Canto II analyzer (BD Biosciences). All analyses were performed using FlowJo X10.0.7r2.

### Xenogeneic mouse models

Male and female 8-12 week old NOD-SCID-IL2rγ-/- (NSG) mice were bred and cared for within the Department of Comparative Medicine at the Mayo Clinic under a breeding protocol approved by the Institutional Animal Care and Use Committee (IACUC). Mice were maintained in an animal barrier spaces that is approved by the institutional Biosafety Committee for BSL2+ level experiments.

### NALM6 cell line xenografts

The CD19+, luciferase+ ALL NALM6 cell line was use to establish ALL xenografts. These xenograft experiments were approved by a different IACUC protocol. Here, 1×10⁶ cells were injected intravenously via a tail vein injection. After injection, mice underwent bioluminescent imaging using a Xenogen IVIS-200 Spectrum camera six days later, to confirm engraftment. Imaging was performed after the intraperitoneal injection of 10 µl/g D-luciferin (15 mg/ml). Mice were then randomized based on their bioluminescent imaging to receive different treatments as outlined in the specific experiments. Typically 1-2×10⁶ CART cells or UTD cells are injected and exact doses are listed in the specific experimental details. Weekly imaging was performed to assess and follow disease burden. Tail vein bleeding was done 7-10 days after injection of CART cells to assess T cell expansion and as needed following that. Mouse peripheral blood was lysed using ACK lysing buffer (Thermofisher) and then used for flow cytometry studies. Bioluminescent images were acquired using a Xenogen IVIS-200 Spectrum camera (PerkinElmer, Hopkinton, MA, USA) and analyzed using Living Image version 4.4 (Caliper LifeSciences, PerkinElmer). For antibody treated mice, antibody therapy (10 mg/kg lenzilumab or isotype control) was commenced IP, for a total of 10 days.

### Primary patient derived ALL xenografts

To establish primary ALL xenografts, NSG mice first received 30 mg/kg busulfan IP. The following day, mice were injected with 2×10⁶ primary blasts derived from the peripheral blood of patients with relapsed refractory ALL. Mice were monitored for engraftment for 4-6 weeks and when CD19+ cells were consistently observed in the blood (>1 cell/µl), they were randomized to receive different treatments of CART19 or UTD (1×10⁶ cells) with or without antibody therapy (10 mg/kg lenzilumab or isotype control IP for a total of 10 days, starting on the day they received CART cell therapy). Mice were periodically monitored for leukemic burden via tail vein bleeding.

### Primary patient derived ALL xenografts for CRS/NT

Similar to the experiments above, mice were IP injected with 30 mg/kg busulfan. The following day, they received 1-2×10⁶ primary blasts derived from the peripheral blood of patients with relapsed refractory ALL. Mice were monitored for engraftment for 4-6 weeks and when CD19+ cell level was high (≥10 cells/µl), they received CART19 (2-5×10⁶ cells) and commenced antibody therapy for a total of 10 days, as indicated in the details of the specific experiment. Mice were weighed on daily basis as a measure of their well-being. Brian MRI of the mice was performed 5-6 days post CART injection and tail vein bleeding was performed 4-11 days post CART injection. Brain MRI images were analyzed using Azalyze.

### MRI acquisition

A Bruker Avance II 7 Tesla vertical bore small animal MRI system (Bruker Biospin) was used for image acquisition to evaluate central nervous system (CNS) vascular permeability. Inhalation anesthesia was induced and maintained via 3 to 4% isoflurane. Respiratory rate was monitored during the acquisition sessions using an MRI compatible vital sign monitoring system (Model 1030; SA Instruments, Stony Brook, NY). Mice were given an IP injection of gadolinium using weight-based dosing of 100 mg/kg, and after a standard delay of 15 min, a volume acquisition T1-weighted spin echo sequence was used (repetition time = 150 ms, echo time = 8 ms, field of view: 32 mm × 19.2 mm × 19.2 mm, matrix: 160 × 96 × 96; number of averages = 1) to obtain T1-weighted images. Gadolinium-enhanced MRI changes were indicative of blood-brain-barrier disruption. Volumetric analysis was performed using Analyze Software package developed by the Biomedical Imaging Resource at Mayo Clinic.

### RNA-Seq on mouse brain tissue

RNA was isolated using miRNeasy Micro kit (Qiagen, Gaithersburg, MD, USA) and treated with RNase-Free DNase Set (Qiagen, Gaithersburg, MD, USA). RNA-seq was performed on an Illumina HTSeq 4000 (Illumina, San Diego, CA, USA) by the Genome Analysis Core at Mayo Clinic. The binary base call data was converted to fastq using Illumina bcl2fastq software. The adapter sequences were removed using Trimmomatic, and FastQC was used to check for quality. The latest human (GRCh38) and mouse (GRCm38) reference genomes were downloaded from NCBI. Genome index files were generated using STAR, and the paired end reads were mapped to the genome for each condition. HTSeq31 was used to generate expression counts for each gene, and DeSeq2 was used to calculate differential expression. Gene ontology was assessed using Enrichr. Figure 16 summarizes the steps detailed above. RNA sequencing data are available at the Gene Expression Omnibus under accession number GSE121591.

### Statistics

Prism Graph Pad and Microsoft Excel used to analyze data. The high cytokine concentrations in the heat map were normalized to "1" and low concentrations normalized to "0" via Prism. Statistical tests described in figure legends.

### Results

### GM-CSF neutralization in vitro enhances CAR-T cell proliferation in the presence of monocytes and does not impair CAR-T cell effector function.

If GM-CSF neutralization after CAR-T cell therapy is to be utilized as a strategy to prevent CRS and NT, it must not inhibit CAR-T cell efficacy. Therefore, our initial experiments aimed to investigate the impact of GM-CSF neutralization on CAR-T cell effector functions. Here, CART19 cells were co-cultured with or without the CD19⁺ALL cell line NALM6 in the presence of lenzilumab (GM-CSF neutralizing antibody) or an isotype control (IgG). We established that lenzilumab, but not IgG control antibody, was indeed able to completely neutralize GM-CSF (Figure 6A) but did not inhibit CAR-T cell antigen specific proliferation (Figure 6B). When CART19 cells were co-cultured with the CD19⁺ cell line NALM6 in the presence of monocytes, lenzilumab in combination with CART19 demonstrated an exponential increase in antigen specific CART19 proliferation compared to CART19 plus isotype control IgG (P<0.0001, Figure 6C). To investigate CAR-T specific cytotoxicity, either CART19 or control UTD T cells were cultured with the luciferase⁺CD19⁺ NALM6 cell line and treated with either isotype control antibody or GM-CSF neutralizing antibody (Figure 1D). GM-CSF neutralizing antibody treatment did not inhibit the ability of CAR-T cells to kill NALM6 target cells (Figure 6D). Overall, these results indicate that lenzilumab does not inhibit CAR-T cell function *in vitro* and enhances CART19 cell proliferation in the presence of monocytes, suggesting that GM-CSF neutralization may improve CAR-T cell mediated efficacy.

### GM-CSF neutralization in vivo enhances CAR-T cell anti-tumor activity in xenograft models.

To confirm that GM-CSF depletion does not inhibit CART19 effector functions, we investigated the role of GM-CSF neutralization with lenzilumab on CART19 antitumor activity in xenograft models. First, a relapse model intended to vigorously investigate whether the antitumor activity of CART19 cells was impacted by GM-CSF neutralization was used. NSG mice were injected with 1×10⁶ luciferase⁺ NALM6 cells and then imaged 6 days later, allowing sufficient time for mice to achieve very high tumor burdens. Mice were randomized to receive a single injection of either CART19 or UTD cells and 10 days of either isotype control antibody or lenzilumab (Figure 7A). GM-CSF assay on serum collected 8 days after CART19 injection revealed that lenzilumab successfully neutralizes GM-CSF in the context of CART19 therapy (Figure 7B). Bioluminescence imaging one week after CART19 injection showed that CART19 in combination with lenzilumab effectively controlled leukemia in this high tumor burden relapse model and significantly better than control UTD cells (Figure 7C). Treatment with CART19 in combination with lenzilumab resulted in potent anti-tumor activity and improved overall survival, similar to CART19 with control antibody despite neutralization of GM-CSF levels, indicating that GM-CSF does not impair CAR-T cell activity *in vivo* (Figure 8). Second, these experiments were performed in a primary ALL patient derived xenograft model, in the presence of human PBMCs as this represents a more relevant heterogeneous model. After conditioning chemotherapy with busulfan, mice were injected with blasts derived from patients with relapsed ALL. Mice were monitored for engraftment for several weeks through serial tail vein bleedings and when the CD19⁺ blasts in the blood were ≥1/µL, mice were randomized to receive CART19 or UTD treatment in combination with PBMCs with either lenzilumab plus an anti-mouse GM-CSF neutralization antibody or isotype control IgG antibodies starting on the day of CART 19 injection for 10 days (Figure 7D). In this primary ALL xenograft model, GM-CSF neutralization in combination with CART19 therapy resulted in a significant improvement in leukemic disease control sustained over time for more than 35 days post CART19 administration as compared to CART19 plus isotype control (Figure 7E). This suggests that GM-CSF neutralization may play a role in reducing relapses and increasing durable complete responses after CART19 cell therapy.

### GM-CSF CRISPR knockout CAR-T cells exhibit reduced expression of GM-CSF, similar levels of key cytokines, and enhanced anti-tumor activity.

To confidently exclude any role for GM-CSF critical in CAR-T cell function, we disrupted the GM-CSF gene during CAR-T cell manufacturing using a gRNA that has been reported to yield high efficiency, cloned into a CRISPR lentivirus backbone. Using this gRNA, we achieved around 60% knockout efficiency in CART19 cells (Figure 9). When CAR-T cells were stimulated with the CD19⁺ cell line NALM6, GM-CSF^{k/o} CAR-T cells produced statistically significantly less GM-CSF compared to CART19 with a wild-type GM-CSF locus ("wild type CART19 cells"). GM-CSF knockout in CAR-T cells did not impair the production of other key T cell cytokines, including IFN-γ, IL-2, or CAR-T cell antigen specific degranulation (CD107a) (Figure 10A) but did exhibit reduced expression of GM-CSF (Figure 10B). To confirm that GM-CSF^{k/o} CAR-T cells continue to exhibit normal functions, we tested their *in vivo* efficacy in the high tumor burden relapsing xenograft model of ALL (as described in Figure 7A). In this xenograft model, utilization of GM-CSF^{k/o} CART19 instead of wild type CART19 markedly reduced serum levels of human GM-CSF at 7 days after CART19 treatment (Figure 10B). Bioluminescence imaging data implied that GM-CSF^{k/o} CART19 cells show enhanced leukemic control compared to CART19 in this model (Figure 11). Importantly, GM-CSF^{k/o} CART19 cells demonstrated significant improvement in overall survival compared to wild type CART19 cells (Figure 10C). Other than GM-CSF, no statistically significantly alterations in either human (Figure 10D) or mouse (Figure 10E) cytokines were detected. Together, these results confirm Figure 6 and 7, indicating that GM-CSF depletion does not impair cytokines that are critical to CAR-T efficacy functions. In addition, the results in Figure 10 indicate that GM-CSF^{k/o} CART may represent a therapeutic option for "built in" GM-CSF control as a modification during CAR-T cell manufacturing.

### Patient derived xenograft model for neurotoxicity and cytokine release syndrome

In this model, conditioned NSG mice were engrafted with primary ALL blasts and monitored for engraftment for several weeks until they developed high disease burden (Figure 12A). When the level of CD19⁺ blasts in the peripheral blood was ≥10/µL, mice were randomized to receive different treatments as indicated (Figure 12A). Treatment with CART19 (with control IgG antibodies or with GM-CSF neutralizing antibodies) successfully eradicated the disease (Fig 12B). Within 4-6 days after treatment with CART19, mice began to develop motor weakness, hunched bodies, and progressive weight loss; symptoms consistent with CRS and NT. This was associated with elevation of key serum cytokines 4-11 days post CART19 injection similar to what is seen in human CRS after CAR-T cell therapy (including human GM-CSF, TNF-α, IFN-γ, IL-10, IL-12, IL-13, IL-2, IL-3, IP-10, MDC, MCP-1, MIP-1α, MIP-1β, and mouse IL-6, GM-CSF, IL-4, IL-9, IP-10, MCP-1, and MIG). These mice treated with CART19 also developed NT as indicated by brain MRI analyses revealing abnormal T1 enhancement, suggestive of blood-brain barrier disruption and possibly brain edema (Figure 12D), together with flow cytometric analysis of the harvested brains revealing infiltration of human CART19 cells (Figure 12E). In addition, RNA-seq analyses of brain sections harvested from mice that developed these signs of NT showed significant upregulation of genes regulating the T cell receptor, cytokine receptors, T cell immune activation, T cell trafficking, and T cell and myeloid cell differentiation (Table 1).

**Table 1. Table of canonical pathways altered in brains from patient derived xenografts after treatment with CART19 cells.**

| **Conical Pathway** | **Adj P-Value** | **Genes** |
|---|---|---|
| **regulation of immune response (GO:0050776)** | 9.45E-14 | IFITM1, ITGB2, TRAC, ICAM3, CD3G, PTPN22, CD3E, ITGAL, SAMHD1, SLA2, CD3D, ITGB7, SLAMF6, B2M, NPDC1, CD96, BTN3A1, ITGA4, SH2D1A, HLA-B, HLA-C, BTN3A2, HLA-A, CD8B, SELL, CD8A, CD226, CD247, CLEC2D, HCST, BIRC3 |
| **cytokine-mediated signaling pathway (GO:0019221)** | 1.36E-12 | IFITM1, SP100, TRADD, ITGB2, IL2RG, SAMHD1, IL27RA, OASL, CNN2, IL18RAP, RIPK1, CCR5, IL12RB1, B2M, GBP1, IL6R, JAK3, CCR2, IL32, ANXA1, IL4R, TGFB1, IL10RB, IL10RA, STAT2, PRKCD, HLA-B, HLA-C, IL16, HLA-A, TNFRSF1B, CD4, IRF3, OAS2, IL2RB, FAS, TNFRSF25, LCP1, P4HB, IL7R, MAP3K14, CD44, IL18R1, IRF9, MYD88, BIRC3 |
| **T cell receptor complex (GO:0042101)** | 1.30E-11 | ZAP70, CD4, CD6, CD8B, CD8A, CD3G, CD247, CD3E, CD3D, CARD11 |
| **T cell activation (GO:0042110)** | 2.07E-11 | ITK, RHOH, CD3G, NLRC3, PTPN22, CD3E, SLA2, CD3D, CD2, ZAP70, CD4, PTPRC, CD8B, CD8A, LCK, CD28, LCP1, LAT |
| **regulation of T cell activation (GO:0050863)** | 2.46E-10 | PTPN22, LAX1, CCDC88B, CD2, CD4, LCK, SIT1, TBX21, TIGIT, JAK3, LAT, PAG1, CCR2 |
| **T cell receptor signaling pathway (GO:0050852)** | 4.35E-08 | ITK, BTN3A1, TRAC, WAS, CD3G, PTPN22, BTN3A2, CD3E, CD3D, ZAP70, CD4, PTPRC, LCK, GRAP2, LCP2, CD247, CARD11, LAT, PAG1 |
| **positive regulation of cytokine production (GO:0001819)** | 1.57502E-07 | GBP5, ANXA1, TGFB1, CYBA, PTPN22, PARK7, TMEM173, CCDC88B, MAVS, CD6, IRF3, CD28, RIPK1, SLAMF6, CD46, IL12RB1, TIGIT, IL6R, CARD11, MYD88, CCR2 |
| **T cell differentiation (GO:0030217)** | 2.36E-07 | ZAP70, CD4, ANXA1, PTPRC, CD8A, LCK, CD28, RHOH, PTPN22, CD3D |
| **cytokine receptor activity (GO:0004896)** | 2.43E-07 | IL4R, IL10RB, IL10RA, IL2RG, CD4, CXCR3, IL2RB, CCR5, IL12RB1, IL7R, IL6R, CD44, CCR2 |
| **type I interferon signaling pathway (GO:0060337)** | 3.27E-07 | IFITM1, SP100, IRF3, OAS2, STAT2, HLA-B, HLA-C, HLA-A, SAMHD1, IRF9, MYD88, OASL |
| **response to cytokine (GO:0034097)** | 0.0004679 | SIGIRR, IFITM1, SP100, HCLS1, RIPK1, PTPN7, IKBKE, IL6R, JAK3, IL18R1, MYD88, AES |
| **regulation of innate immune response (GO:0045088)** | 0.001452 | GBP5, GFI1, STAT2, ADAM8, NLRC3, PTPN22, SAMHD1, BIRC3 |
| **regulation of tumor necrosis factor production (GO:0032680)** | 0.003843 | CD2, MAVS, CYBA, NLRC3, PTPN22, RIPK1, SLAMF1 |
| **T cell receptor binding (GO:0042608)** | 0.0102397 | LCK, CD3G, CD3E |
| **regulation of tumor necrosis factor-mediated signaling pathway (GO:0010803)** | 0.0124059 | SHARPIN, TRADD, CASP4, RIPK1, TRAF1, BIRC3 |
| **positive regulation of myeloid leukocyte differentiation (GO:0002763)** | 0.0376647 | CD4, HCLS1, RIPK1, EVI2B |

### GM-CSF neutralization in vivo ameliorates cytokine release syndrome and neurotoxicity after CART19 therapy in a xenograft model.

Using the xenograft patient derived model for NT and CRS shown in Figure 4A, we investigated the effect of GM-CSF neutralization on CART19 toxicities. To rule out the cofounding effect of mouse GM-CSF, mice received CART19 cells in combination with 10 days of GM-CSF antibody therapy (10mg/kg lenzilumab and 10mg/kg anti-mouse GM-CSF neutralizing antibody) or isotype control antibodies. GM-CSF neutralizing antibody therapy prevented CRS induced weight loss after CART19 therapy (Figure 13A). Cytokine analysis 11 days after CART19 cell therapy showed that human GM-CSF was neutralized by the antibody (Figure 13B). In addition, GM-CSF neutralization resulted in significant reduction of several human (IP-10, IL-3, IL-2, IL-IRa, IL-12p40, VEGF, GM-CSF) (Figure 5C) and mouse (MIG, MCP-1, KC, IP-10) (Figure 13D) cytokines. Interferon gamma-induced protein (IP-10, CXCL10) is produced by monocytes among other cell types and serves as a chemoattractant for numerous cell types including monocytes, macrophages, and T cells. IL-3 plays a role in myeloid progenitor differentiation. IL-2 is a key T cell cytokine. Interleukin-1 receptor antagonist (IL-IRa) inhibits IL-1. (IL-1 is produced by macrophages and is a family of critical inflammatory cytokines.) IL-12p40 is a subunit of IL-12, which is produced by macrophages among other cell types and can encourage Th1 differentiation. Vascular endothelial growth factor (VEGF) encourages blood vessel formation. Monokine induced by gamma interferon (MIG, CXCL9) is a T cell chemo attractant. Monocyte chemoattractant protein 1 (MCP-1, CCL2) attracts monocytes, T cells, and dendritic cells. KC (CXCL1) is produced by macrophages among other cell types and attracts myeloid cells such as neutrophils. There was also a trend in reduction of several other human and moue cytokines after GM-CSF neutralization. This suggests that GM-CSF plays a role in the downstream activity of several cytokines that are instrumental in the cascade that results in CRS and NT.

Brain MRIs 5 days after CAR19 treatment showed that GM-CSF neutralization reduced T1 enhancement as a measure of brain inflammation, blood-brain barrier disruption, and possibly edema, compared to CART19 plus control antibodies. The MRI images after GM-CSF neutralization (with lenzilumab and anti-mouse GM-CSF antibody) were similar to baseline pre-treatment scans, suggesting that GM-CSF neutralization effectively helped abrogated the NT associated with CART19 therapy (Figure 14A, B). Using human ALL blasts and human CART19 in this patient-derived xenograft model, GM-CSF neutralization after CART19 reduced neuro-inflammation by 59% compared to CART19 plus isotype controls (Figure 14B). This is a significant finding, and the first time it has been demonstrated *in vivo* that the NT caused by CART19 can be effectively abrogated. Human CD3 T cells were present in the brain after CART19 therapy as assayed by flow cytometry, and with GM-CSF neutralization, there was a trend toward reduction in brain CD3 T cells (Figure 14C). Finally, a trend in reduction of CD11b+ bright macrophages was observed in the brains of mice receiving GM-CSF neutralization during CAR-T cell therapy compared to isotype control during CAR-T therapy (Figure 14D), implicating that GM-CSF neutralization helps reduce macrophages within the brain.

### Example 3: GM-CSF interaction with GM-CSF receptors

### Materials and Methods

### GM-CSF Receptor (CSF2R) Analysis

T Cell Expansion. Isolated T cells were stimulated with CD3/CD28 beads. Expression of CSF2 Receptors CSF2RA (CD116) and CSF2RB (CD131) was measured by flow cytometry on days 0, 1, 3, 6, and 8. Resting T cells were used as the negative control.

CART Production. CART19 was produced, and CSF2RA (CD116) and CSF2RB (CD131) expression was measured by flow cytometry on days 0, 1, 3, and 6. Resting T cells were used as a negative control, and the Nalm6 cell line was used for a positive control.

### Western Blot

CART19/UTD cells and irradiated Nalm6 cells were co-cultured at a 1:1 ratio. Antibodies were added to the cells at a dose of 10 µg/mL. The culture conditions were UTD, CART19, CART19 + GM-CSF blockade, CART19 + CSF2RA blockade, and CART19 + CSF2RB blockade. These conditions were tested with a Media control versus Nalm6 stimulation

After culturing the cells with antibodies for 24 hours, CART19 were isolated using microbeads. CART19 purity (98-100%) was verified using flow cytometry. Cells were collected by spinning down a cell pellet, and polypeptides were isolated from the cells for use in western blotting.

### Results

GM-CSF receptors were upregulated on T cells and CART cells upon stimulation. Levels of GM-CSF receptors CSF2RA (CD116) and CSF2RB (CD131) on T cells were measured and compared to levels of CSF2RA (CD116) and CSF2RB (CD131) on resting T cells (negative control) during an 8-day T cell expansion protocol. CSF2RA and CSF2RB expression increased after initial stimulation, peaked on Day 3, and slightly reduced after debeading on Day 6 (Figure 17A). Levels of CSF2RA (CD116) and CSF2RB (CD131) were also measured on CART19 and UTD cells and compared to levels of CSF2RA (CD116) and CSF2RB (CD131) on control cells during an 8-day CART production. Expression decreases slightly on Day 1 but peaks on Day 3 (Figure 17B).

GM-CSF interaction with CSF2 Receptor depends on the beta chain (CSF2RB). Phosphorylated Stat5 and phosphorylated Jak2 protein expression increased in the presence of irradiated Nalm6 and CSF2RA blockade but decreased in the presence of GM-CSF and CSF2RB blockade (Figure 18). FAS is downstream of the CSF2 receptor pathway and its expression is slightly decreased in the presence of GM-CSF blockade with Nalm6 but not in the presence of CSF2RA or in the presence of CSF2RB blockade (Figure 18).

These results demonstrate that CSF2R can be expressed on activated T cells and CART cells. These results also demonstrate that GM-CSF neutralization can inhibit p-STATS. A similar inhibition was observed when GM-CSF receptor beta was blocked, suggesting that the signaling is driven by interaction between GM-CSF and GM-CSF receptor beta on activated CART cells.

### Example 4: GM-CSF and CART19 cell transcription

### Materials and Methods

### RNA-Seq

Isolation of RNA and RNA-Seq. Total RNA was isolated from three biological replicates (normal donors 105, 115, and 116) for untransduced T cells, CART19, and GM-CSF^{k/o} CART19 using miRNeasy Micro kit (QIAGEN) and treated with RNase-Free DNase Set (QIAGEN). Paired-end RNA-seq was performed on an Illumina HTSeq 4000 by the Genome Analysis Core at Mayo Clinic.

Quality. The binary base call data was converted to fastq using Illumina bcl2fastq software. The adapter sequences were removed using Trimmomatic. FastQC was used to check quality.

Alignment. The latest human reference genome was downloaded from NCBI (HG38). Genome index files were generated using STAR, and the paired end reads were mapped to the genome for each condition.

Differential Expression. HTSeq was used to generate expression counts for each gene, and DeSeq2 was used to calculate differential expression. Conditions with less than 10 total transcripts were filtered out. An adjusted p-value between GM-CSF^{k/o} CART19 and CART19 was calculated using the Benjamini-Hochberg method.

### Results

RNA-Seq was used to identify transcriptome differences between GM-CSF^{k/o} CART19 cells and CART19 cells on Day 8 of CART production. 236 genes were identified that are significantly differentially expressed with a Benjamini-Hochberg adjusted p-value <0.05 (Figure 19A). Three distinct gene expression patterns were identified: genes that were upregulated in GM-CSF^{k/o} CART19 cells as compared to CART19 cells and UTD cells (Figure 19A, top); genes that were downregulated in GM-CSF^{k/o} CART19 cells as compared to CART19 cells and UTD cells (Figure 19A, middle); and genes that were normalized to the level in UTD cells in GM-CSF^{k/o} CART19 cells as compared to CART19 cells (Figure 19A, bottom). Among the genes in the middle profile is FAS, which is a part of the Tec Kinase Pathway and responsible for inducing apoptosis. Genes were also identified that were significantly downregulated in GM-CSF^{k/o} CART19 cells as compared to CART19 cells (Figure 19B). Notably, GM-CSF knockout in CART19 cells normalized the differential gene expression seen in the CART19 cells (Figure 19C). Principle component analysis shows the GM-CSF^{k/o} CART19 cells overall gene expression pattern is more like UTD cells than CART19 cells.

These results demonstrate that GM-CSF^{k/o} cells can have a distinct gene expression pattern with an increase in downregulated genes in GM-CSF^{k/o} CART19 cells.

### Example 5: GM-CSF editing by CRISPR-Cas9

### Materials and Methods

### Whole exome sequencing (WES)

Sample Processing. DNA was isolated from three biological replicates each of untransduced T cells, GM-CSF^{k/o} T cells, CART19 cells, and GM-CSF^{k/o} CART19 cells using PureLink Genomic DNA Mini Kit. Extracted DNA was submitted to the Mayo Clinic Medical Genome Facility Genome Analysis Core for WES. The samples were sequenced on an Illumina HiSeq 4000.

Primary Analysis. Primary analysis was performed by the Core by converting the binary base call data was to fastq using Illumina bcl2fastq software.

Secondary Analysis. The fastq files were aligned against the human reference genome HG38 using BWA-Mem aligner. Secondary analysis was performed by the Division of Biomedical Statistics and Informatics by using Genome Analysis ToolKit (GATK) to call variants and generate raw VCF data.

Off Target Candidates. SAS 9.4 custom code was used to find differences in editing between the samples. Specific editing in the CSF2 target gene was first investigated. Next, variants found in the GM-CSF^{k/o} conditions but not their controls (untransduced T cells and CART19) were compared, excluding variants that were inherent to the individual's genome (present in the untransduced T cells). These lists were then cross-referenced and the candidate CRISPR/Cas9 edits were classified as T Cell only, CART19 only, or Both T Cell and CART19. Alternate allele frequencies of <10%, SNPs with a read depth of <10, and samples that did not pass the quality filter (VQSQRT analysis) were excluded. This was depicted using the VennDiagram package in R.

Off Target Predictions. Three different off-target editing prediction tools were used: Cas-OFFinder, CRISTA, and CCTop. The 15,632 predictions generated by Cas-OFFinder (query sequence settings: ≤ 6 mismatches, DNA/RNA bulge size ≤ 2) included all predictions generated by CRISTA or CCTop. As such, only Cas-OFFinder predictions were used for analysis. The candidate CRISPR/Cas9 edits were compared to the CAS-OFFinder off-target predictions by matching the variant position and chromosome.

Genomic Prevalence. The CRISPR/Cas9 edited candidate list was cross-referenced with previously generated RNA-Seq data on the same biological replicates. The candidate list was also filtered by genomic prevalence as defined by the 1000 Genomes Project (allele frequency ≤1% was considered rare).

Hypothesis Testing. The number of single nucleotide variants (substitutions) or indels (insertions or deletions) in untransduced T cells was compared to knockout T cells, the number in CART19 cells was compared to knockout CART19 cells, and the number in all controls was compared to all knockout cells. The differences in single nucleotide variants and indels were depicted by violin plot using GraphPad Prism version 8.1.1 for Windows. Because the samples are dependent, the Wilcoxon signed-rank test was used.

### Results

WES was used to identify precise CSF2 gene editing locations by CRISPR-Cas9. A CSF2 CRISPR gRNA 1 (SEQ ID NO:7) was expected to have a cut site 3 bp upstream of the PAM site (Figure 20A, top panel). The actual cut site was determined to be 6 bp upstream of the PAM site, and could result in insertions and deletions at base 132074828 of chromosome 5 schema (Figure 20A, bottom panel). The difference in Cas9 cut site may be due to the adjacent PAM site on the reverse strand. The frequency of insertions and deletions in each biological replicate of CART19 is shown in Figure 20A, bottom panel.

Single nucleotide variant (SNV) counts and insertion/deletion (indel) counts in the CRISPR knockout conditions as compared to their controls (T Cells and CART19 cells) were identified (Figure 20B). No significant difference was found between groups (Wilcoxon signed-rank test, p-value = 0.16).

A representation of the total variants found in CRISPR-edited cells versus their respective control (CART19 or T Cells) was also identified. The SNPs were pre-filtered for genomic prevalence in the population (less than 1% in 1000 Genomes Project) and for presence in all three biological replicates. 0.004% (4) of the SNPs were found in CART19 cells only, 0.006% (5) of the SNPs were found in T Cells only, 0.0001% (1) of the SNPs was found in both CART19 cells and T Cells, and 99% (12,439) of the SNPs were filtered out (Figure 20C). The SNP present in both T-cell and CART19 cells (in the intersection of Figure 20C) was identified as the deletion in the CSF2 gene shown in the bottom panel of Figure 20A.

These results demonstrate that the only edit that matched the CasOFFinder off-target predictions was the CSF2 edit, and that GM-CSF disruption in CART19 cells by CRISPR/Cas9 is safe method of knocking out GM-CSF.

### Example 6: GM-CSF and CART19 monocyte differentiation

### Materials and Methods

PBMCs were isolated, and monocytes were separated with Classical Monocyte Isolation Kit (Miltenyi Biotec). Then, monocytes were differentiated into M1 macrophages or M2 macrophages with CellXVivo Human M1 or M2 Macrophage Differentiation Kit (R&D Systems).

CART19 cells, Nalm6, and M1 macrophages or M2 macrophages were co-cultured in 1:1:1 ratio. Cells were harvested at day 3, and were stained for CD3 and Live Dead aqua (Invitrogen). Countbright beads (Invitrogen) were added prior to flow cytometric analysis for absolute quantification.

### Results

Neutralizing GM-CSF in the presence of M1 macrophages did not statistically significantly alter CART19 expansion upon CD19 stimulation; however, there was a trend toward enhanced proliferation of CAR-T cell with GM-CSF neutralization. GM-CSF blockade statistically significantly enhanced CART19 expansion upon CD19 stimulation in the presence of M2 macrophages (Figure 21).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method for making a chimeric antigen receptor T cell having a reduced level of granulocyte-macrophage colony-stimulating factor (GM-CSF) polypeptides, said method comprising:
introducing a nucleic acid construct into a T cell ex vivo, wherein said nucleic acid construct comprises: a) a nucleic acid encoding a guide RNA, wherein said guide RNA is complementary to a GM-CSF messenger RNA; b) a nucleic acid encoding a Cas nuclease, and c) a nucleic acid encoding said chimeric antigen receptor,
wherein the nucleic acid encoding the chimeric antigen receptor comprises the nucleic acid sequence set forth in SEQ ID NO: 2 and wherein the chimeric antigen receptor targets CD19 tumor-associated antigen.

2. The method of claim 1, wherein said guide RNA comprises a nucleic acid sequence set forth in SEQ ID NO:1.

3. The method of any of claims 1 or 2, wherein said Cas nuclease is Cas9 nuclease.

4. The method of any of claims 1 to 3, wherein said nucleic acid construct is a viral vector.

5. The method of claim 4, wherein said viral vector is a lentiviral vector.

6. The method of any of claims 1 to 5, wherein said introducing step comprises transduction.

7. A chimeric antigen receptor T cell having a reduced level of granulocyte-macrophage colony-stimulating factor (GM-CSF) polypeptides for use in the treatment of cancer, wherein the chimeric antigen receptor T cell comprises a chimeric antigen receptor encoded by the nucleic acid sequence set forth in SEQ ID NO: 2 and wherein the chimeric antigen receptor targets CD19 tumor-associated antigen.

8. The chimeric antigen receptor T cell for use according to claim 7, wherein said cancer is a lymphoma.

9. The chimeric antigen receptor T cell for use according to claim 8, wherein said lymphoma is a diffuse large B cell lymphoma.

10. The chimeric antigen receptor T cell for use according to claim 7, wherein said cancer is a leukemia.

11. The chimeric antigen receptor T cell for use according to claim 10, wherein said leukemia is an acute lymphoblastic leukemia.

12. A method for improving T cell effector functions of a chimeric antigen receptor T cell, said method comprising:
introducing a nucleic acid construct into a T cell ex vivo, wherein said nucleic acid construct comprises: a) a nucleic acid encoding a guide RNA, wherein said guide RNA is complementary to a GM-CSF messenger RNA; b) a nucleic acid encoding a Cas nuclease, and c) a nucleic acid encoding said chimeric antigen receptor,
wherein the nucleic acid encoding the chimeric antigen receptor comprises the nucleic acid sequence set forth in SEQ ID NO:2 and wherein the chimeric antigen receptor targets CD19 tumor-associated antigen.

## Patentansprüche

1. Verfahren zur Herstellung einer chimären Antigen-Rezeptor-T-Zelle, die ein reduziertes Niveau von Granulozyten-Makrophagen-Kolonie-stimulierendem Faktor (GM-CSF)-Polypeptiden hat, wobei das Verfahren wie folgt umfasst:
Einführen eines Nukleinsäurekonstrukts in eine T-Zelle ex vivo, wobei das Nukleinsäurekonstrukt wie folgt umfasst: a) eine Nukleinsäure, die für eine Guide-RNA codiert, wobei die Guide-RNA komplementär zu einer GM-CSF-Boten-RNA ist; b) eine Nukleinsäure, die für eine Cas-Nuklease codiert und c) eine Nukleinsäure, die für den chimären Antigen-Rezeptor codiert,
wobei die Nukleinsäure, die für den chimären Antigen-Rezeptor codiert, die in SEQ ID NO: 2 enthaltene Nukleinsäuresequenz umfasst und wobei der chimäre Antigen-Rezeptor das mit CD19 tumorassoziierte Antigen anzielt.

2. Verfahren nach Anspruch 1, wobei die Guide-RNA eine in SEQ ID NO: 1 enthaltene Nukleinsäuresequenz umfasst.

3. Verfahren nach Anspruch 1 oder 2, wobei die Cas-Nuklease Cas9-Nuklease ist.

4. Verfahren nach einem der Ansprüche 1 bis 3, wobei das Nukleinsäurekonstrukt ein viraler Vektor ist.

5. Verfahren nach Anspruch 4, wobei der virale Vektor ein Lentivirus-Vektor ist.

6. Verfahren nach einem der Ansprüche 1 bis 5, wobei der Einführungsschritt eine Transduktion umfasst.

7. Chimäre Antigen-Rezeptor-T-Zelle, die ein reduziertes Niveau von Granulozyten-Makrophagen-Kolonie-stimulierendem Faktor (GM-CSF)- Polypeptiden hat, zur Verwendung bei der Krebsbehandlung, wobei die chimäre Antigen-Rezeptor-T-Zelle einen chimären Antigen-Rezeptor, für den die in SEQ ID NO: 2 enthaltene Nukleinsäuresequenz codiert, umfasst und wobei der chimäre Antigen-Rezeptor das mit CD19 tumorassoziierte Antigen anzielt.

8. Chimäre Antigen-Rezeptor-T-Zelle zur Verwendung nach Anspruch 7, wobei die Krebserkrankung ein Lymphom ist.

9. Chimäre Antigen-Rezeptor-T-Zelle zur Verwendung nach Anspruch 8, wobei das Lymphom ein diffuses großzelliges B-Zell-Lymphom ist.

10. Chimäre Antigen-Rezeptor-T-Zelle zur Verwendung nach Anspruch 7, wobei die Krebserkrankung Leukämie ist.

11. Chimäre Antigen-Rezeptor-T-Zelle zur Verwendung nach Anspruch 10, wobei die Leukämie eine akute lymphoblastische Leukämie ist.

12. Verfahren zum Verbessern von T-Zell-Effektorfunktionen einer chimären Antigen-Rezeptor-T-Zelle, wobei das Verfahren wie folgt umfasst:
Einführen eines Nukleinsäurekonstrukts in eine T-Zelle ex vivo, wobei das Nukleinsäurekonstrukt wie folgt umfasst: a) eine Nukleinsäure, die für eine Guide-RNA codiert, wobei die Guide-RNA komplementär zu einer GM-CSF-Boten-RNA ist; b) eine Nukleinsäure, die für eine Cas-Nuklease codiert und c) eine Nukleinsäure, die für den chimären Antigen-Rezeptor codiert,
wobei die Nukleinsäure, die für den chimären Antigen-Rezeptor codiert, die in SEQ ID NO: 2 enthaltene Nukleinsäuresequenz umfasst und wobei der chimäre Antigen-Rezeptor das mit CD19 tumorassoziierte Antigen anzielt.

## Revendications

1. Procédé de fabrication d'un lymphocyte T récepteur d'antigène chimérique ayant un niveau réduit de polypeptides du facteur de stimulation des colonies de granulocytes-macrophages (GM-CSF), ledit procédé comprenant :
l'introduction d'une construction d'acide nucléique dans un lymphocyte T *ex vivo,* dans laquelle ladite construction d'acide nucléique comprend : a) un acide nucléique codant pour un ARN guide, ledit ARN guide étant complémentaire d'un ARN messager du GM-CSF ; b) un acide nucléique codant pour une nucléase Cas, et c) un acide nucléique codant pour ledit récepteur d'antigène chimérique,
dans lequel l'acide nucléique codant pour le récepteur d'antigène chimérique comprend la séquence d'acide nucléique présentée dans SEQ ID NO : 2 et dans laquelle le récepteur d'antigène chimérique cible l'antigène associé à la tumeur CD19.

2. Procédé selon la revendication 1, dans lequel ledit ARN guide comprend une séquence d'acide nucléique présentée dans SEQ ID NO : 1.

3. Procédé selon l'une quelconque des revendications 1 ou 2, dans lequel ladite nucléase Cas est la nucléase Cas9.

4. Procédé selon l'une quelconque des revendications 1 à 3, dans lequel ladite construction d'acide nucléique est un vecteur viral.

5. Procédé selon la revendication 4, dans lequel ledit vecteur viral est un vecteur lentiviral.

6. Procédé selon l'une quelconque des revendications 1 à 5, dans lequel ladite étape d'introduction comprend une transduction.

7. Lymphocyte T récepteur d'antigène chimérique ayant un niveau réduit de polypeptides du facteur de stimulation des colonies de granulocytes-macrophages (GM-CSF) pour une utilisation dans le traitement du cancer, le lymphocyte T récepteur d'antigène chimérique comprenant un récepteur d'antigène chimérique codé par la séquence d'acide nucléique présentée dans SEQ ID NO : 2 et le récepteur d'antigène chimérique ciblant l'antigène associé à la tumeur CD19.

8. Lymphocyte T récepteur d'antigène chimérique pour une utilisation selon la revendication 7, ledit cancer étant un lymphome.

9. Lymphocyte T récepteur d'antigène chimérique pour une utilisation selon la revendication 8, ledit lymphome étant un lymphome diffus à grandes cellules B.

10. Lymphocyte T récepteur d'antigène chimérique pour une utilisation selon la revendication 7, ledit cancer étant une leucémie.

11. Lymphocyte T récepteur d'antigène chimérique pour une utilisation selon la revendication 10, ladite leucémie étant une leucémie aiguë lymphoblastique.

12. Procédé pour améliorer les fonctions effectrices des lymphocytes T d'un lymphocyte T récepteur d'antigène chimérique, ledit procédé comprenant :
l'introduction d'une construction d'acide nucléique dans un lymphocyte T *ex vivo,* ladite construction d'acide nucléique comprenant : a) un acide nucléique codant pour un ARN guide, ledit ARN guide étant complémentaire d'un ARN messager GM-CSF ; b) un acide nucléique codant pour une nucléase Cas, et c) un acide nucléique codant pour ledit récepteur d'antigène chimérique, l'acide nucléique codant pour le récepteur d'antigène chimérique comprenant la séquence d'acide nucléique présentée dans SEQ ID NO : 2 et le récepteur d'antigène chimérique ciblant l'antigène associé à la tumeur CD19
